# EUROPEAN PATENT SPECIFICATION

(11) **EP 1 480 679 B1**
(45) Date of publication and mention of the grant of the patent: **23.05.2007**
(21) Application number: 03706732.9
(22) Date of filing: 24.02.2003
(51) Int. Cl.: A61K 47/38, A61K 31/5377, A61P 35/00

(54) **PHARMACEUTICAL FORMULATION OF IRESSA COMPRISING A WATER-SOLUBLE CELLULOSE DERIVATIVE**
PHARMAZEUTISCHE IRESSA ZUSAMMENSETZUNG ENTHALTEND EIN WASSERLÖSLICHES CELLULOSEDERIVAT
FORMULATION PHARMACEUTIQUE D'IRESSA COMPRENANT UN DERIVE DE CELLULOSE SOLUBLE DANS L'EAU

(30) Priority: 26.02.2002 GB 0204392; 30.05.2002 GB 0212462; 11.06.2002 GB 0213267
(43) Date of publication of application: 01.12.2004
(73) Proprietor: AstraZeneca AB, 151 85 (SE)
(72) Inventor: GELLERT, Paul Richard, Macclesfield, Cheshire SK10 4TG (GB); PARKER, Michael Davis, Macclesfield, Cheshire SK10 2NA (GB); DE MATAS, Marcel, Macclesfield, Cheshire SK10 4TG (GB)
(86) International application number: PCT/GB2003/000803
(87) International publication number: WO 2003/072139

(56) References cited:
- WO-A-96/33980
- GB-A- 2 306 885
- US-A- 4 344 934
- US-A1- 2003 045 537

## Description

The present invention relates to pharmaceutical compositions, more particularly to oral pharmaceutical compositions containing 4-(3'-chloro-4'-fluoroanilino)-7-methoxy-6-(3-morpholinopropoxy)quinazoline or a pharmaceutically-acceptable salt thereof (hereinafter referred to as the "Agent").

The Agent is disclosed in International Patent Application WO 96/33980 (Example 1) and is a potent inhibitor of the epidermal growth factor receptor (EGFR) family of tyrosine kinase enzymes such as erbB1. The Agent has the structure of the Formula I 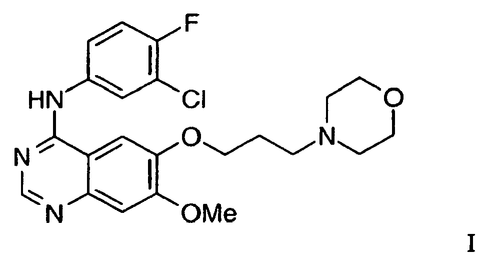 and is now known as Iressa (registered trade mark), gefitinib (Unites States Adopted Name), by way of the code number ZD1839 and Chemical Abstracts Registry Number 184475-35-2.

The Agent possesses anti-proliferative activity such as anti-cancer activity and, accordingly, is useful in methods of treatment of proliferative disease such as cancer in the human or animal body. The Agent is expected to be useful in the treatment of diseases or medical conditions mediated alone or in part by EGF (particularly erbB 1) receptor tyrosine kinases, particularly cancers such as lung (especially non-small cell lung cancer), breast, prostate, ovarian, colorectal, gastric, brain, head and neck, bladder, pancreas, oesophageal, stomach, renal, skin, gynaecological and thyroid cancers and in the treatment of a range of leukaemias, lymphoid malignancies and solid tumours such as carcinomas and sarcomas. The Agent has recently undergone Phase III trials for the treatment of non-small cell lung cancer.

The Agent is a weakly basic compound and has two basic groups with pKₐ's of 5.3 and 7.2. The protonation and deprotonation of these basic groups has a marked effect upon the solubility of the Agent in aqueous media. Consequently, the solubility of the Agent is highly dependent upon pH. For example, the free-base form of the Agent is soluble at pH 1 (10 to 30 ml of aqueous solvent required to dissolve 1g of Agent) but is practically insoluble above pH 7, with the solubility dropping sharply between pH 4 and pH 6 (≥10000 ml of aqueous solvent required to dissolve 1g of Agent at pH 6).

Compounds which have pH dependent solubility, particularly basic compounds, may exhibit undesirable pharmacokinetic properties such as problems in their absorption, possibly producing low or variable bioavailability between patients and between doses.

A factor which can affect the absorption of an orally administered drug is the changing pH experienced by the drug as it passes through the GI tract. Typically a drug may be absorbed in a number of different sites along the GI tract following oral administration for example, the cheek lining, stomach, duodenum, jejunum, ileum and colon. The pH may be different at each site of absorption with the pH significantly different from the stomach (pH 1-3.5) to the small intestine (pH 4-8). When the solubility of a drug varies with pH the drug may precipitate from solution as it passes through the GI tract. This can result in variability in the extent and/or rate of absorption between doses and between patients, because the drug needs to be in solution to be absorbed.

Although the Agent has a high solubility in the acidic environment of the stomach, it is not significantly absorbed from this area. The site of highest intrinsic absorption for the Agent is thought to be the upper intestine. However, in this region of the GI tract the pH is relatively high compared to that in the stomach and the Agent has a reduced solubility at the higher pH. As a result the Agent is prone to precipitate from solution as it passes from the acidic environment of the stomach to the higher pH environment of the upper GI tract (such as the upper intestine), resulting in reduced and/or variable absorption of the Agent. Furthermore, in view of the particular pH sensitivity of the Agent, even small variations in local pH may have a significant effect upon its pharmacokinetic profile. The pH of the GI tract can also vary as a result of, for example, whether a patient is in a fed or fasted state and the rate of gastric emptying. The combination of the sensitive pH solubility profile of the Agent together with the variability of the pH in the GI tract may result in a high degree of inter-patient variability in the bioavailability and/or plasma concentrations of the Agent and possibly sub-optimal treatment efficacy in a proportion of patients. There is therefore a need to improve the pharmacokinetic properties of the Agent.

US 4,344,934 describes a pharmaceutical composition comprising wetted mixtures of a poorly water-soluble drug and a water-soluble polymer which are stated to exhibit improved bio-availability.

GB 2,306,885 describes a topical composition containing a drug with pH dependent solubility, wherein the composition becomes supersaturated with the drug when the composition is applied to the skin as a result of the change in pH. The compositions optionally contain an anti-nucleating agent to inhibit precipitation of the drug from the composition.

Usui et al (Int. J. Pharmaceutics 154 (1997) 59-66) found that certain water-soluble polymers inhibited the precipitation of a specific compound, RS-8359, from supersaturated aqueous methanol solutions.

Loftsson et al (Int. J. Pharmaceutics 127 (1996) 293-296) describe the effects of water-soluble polymers on drug solubility of the compounds acetazolamide, hydrocortisone, prazepam and sulfamethoxazole.

We have surprisingly found that the rate at which the Agent is precipitated from solution when the pH of the solution increases from a pH similar to that of the stomach to a pH similar that found in the upper GI tract (such as the upper intestine), is significantly reduced when the Agent is formulated or administered together with certain excipients. This is expected to provide improved pharmacokinetic properties for example, increased absorption and/or bioavailability and may reduce inter-patient variability in the bioavailability and/or plasma concentration of the Agent, because the Agent remains in solution for longer in the region of the GI tract with the highest intrinsic absorption rates for the Agent.

According to a first aspect of the present invention there is provided a pharmaceutical composition comprising the Agent and a water-soluble cellulose ether or an ester of a water-soluble cellulose ether.

According to another aspect of the present invention there is provided a pharmaceutical composition comprising the Agent and a water-soluble cellulose ether.

According to another aspect of the present invention there is provided a pharmaceutical composition comprising the Agent and an ester of a water-soluble cellulose ether.

By "cellulose ether" is meant an ether formed by the conversion of one or more hydroxy groups present on one or more of the anhydroglucose repeat unit of a cellulose polymer to provide one or more ether linked group on the cellulose polymer. By way of example, suitable ether linked groups that may be present on anhydroglucose repeat units of the cellulose polymer include (1-4C)alkyl optionally substituted by one or more substituent(s) selected from hydroxy, carboxy, (1-4C)alkoxy and hydroxy(1-4C)alkoxy. Particular ether linked groups include, for example, (1-4C)alkyl such as methyl or ethyl; hydroxy(1-4C)alkyl, such as 2-hydroxyethyl, 2-hydroxypropyl or 3-hydroxypropyl; (1-4C)alkoxy(1-4C)alkyl, such as 2-methoxyethyl, 3-methoxypropyl, 2-methoxypropyl or 2-ethoxyethyl; hydroxy(I-4C)alkoxy(1-4C)alkyl such as 2-(2-hydroxyethoxy)ethyl or 2-(2-hydroxypropoxy)propyl; carboxy(1-4C)alkyl, such as carboxymethyl; or groups of the formula H-[O-(1-4C)alkyl-]ₘ, wherein m=1 to 5, for example 1, 2 or 3, such as H-[O-CH(CH₃)CH₂-]ₘ or H-[O-CH₂CH₂-]ₘ. For the avoidance of any doubt, the term "ether-linked groups" refers to one or more of the above groups linked to the cellulose polymer by an oxygen atom. For example where the ether linked group is methyl, one or more of the hydroxy groups of the anhydroglucose repeat units are converted to methoxy.

The water-soluble cellulose ether may carry the same ether linked groups, for example methyl groups as in the case of methylcellulose. Alternatively the water-soluble cellulose ether may carry a plurality of different ether-linked groups. For example hydroxypropyl methylcellulose refers to a cellulose which carries both methyl and hydroxypropyl (for example 2-hydroxypropyl) ether linked groups.

By "water-soluble cellulose ether" is meant cellulose ethers that dissolve or disperse in water to give a colloidal solution or dispersion at a temperature of less than 30°C (for example from 10 to 20°C). Generally the water-soluble cellulose ethers will have a solubility in water of at least 20mg/ml, suitably at least 30mg/ml at a temperature of 10 to 20°C (wherein the solubility is determined in un-buffered distilled water). Suitable water-soluble cellulose ethers include, those listed in the Handbook of Pharmaceutical Excipients, 3^{rd} Edition American Pharmaceutical Association, for example methylcellulose, hydroxyethylcellulose, hydroxypropylcellulose, hydroxypropyl methylcellulose, hydroxybutyl methylcellulose, hydroxyethyl ethylcellulose, a water-soluble salt of carboxymethylcellulose (for example sodium carboxymethylcellulose) and a water-soluble salt of carboxymethyl hydroxyethyl cellulose (for example sodium carboxymethyl hydroxyethylcellulose). More particularly a suitable water-soluble cellulose ether is selected from, for example, hydroxyethylcellulose, hydroxypropylcellulose, hydroxypropyl methylcellulose, and a water-soluble salt of carboxymethylcellulose (for example sodium carboxymethyl cellulose).

In one embodiment of the present invention the water-soluble cellulose ether is hydroxypropyl methylcellulose (HPMC). As mentioned hereinbefore the cellulose polymer backbone of HPMC carried both methoxy and hydroxypropoxy (particularly 2-hydroxypropoxy) groups. A wide range of grades of HPMC may be used for example with a dynamic viscosity of < 60cP, such as from 2 to 18, suitably from 5 to 7cP, wherein the dynamic viscosity is measured in a 2%w/v aqueous solution of the HPMC at 20°C. The HPMC suitably has a degree of substitution of from 10 to 35% (suitably from 25 to 35%) methoxy groups and 3-30% (suitably from 5 to 15%) hydroxypropoxy groups. Unless specified otherwise the term "% degree of substitution" used herein refers to the average % by weight of methoxy and hydroxypropoxy groups based upon the dry weight of the water-soluble cellulose ether (eg HPMC). Particular grades of HPMC include 2910, 1828, 2208 and 2906 (wherein the first two digits refer to the average degree of methoxy substitution and the second two digits to the average degree of hydroxypropoxy substitution) as described in the Handbook of Pharmaceutical Excipients, 3^{rd} Edition 2000 American Pharmaceutical Association p 252. More particularly, the above grades of HPMC with a dynamic viscosity of from 2 to 18 cP (for example from 5 to 7cP). Still more particularly the HPMC is grade 2910 with a dynamic viscosity of from 5 to 7 cP, wherein the dynamic viscosity is measured in a 2%w/v aqueous solution of the HPMC at 20°C.

Unless specified otherwise; the term dynamic viscosity used herein refers to viscosity measurement at the temperature quoted using a suitable apparatus such as a Brookfield viscometer fitted with a #2 spindle and a rotation rate of 60 rpm.

In another embodiment of the present invention the water-soluble cellulose ether is not hydroxypropyl methylcellulose.

In a further embodiment of the present invention the water-soluble cellulose ether is selected from hydroxyethylcellulose and hydroxypropylcellulose. When the water-soluble cellulose ether is a hydroxypropylcellulose the degree of substitution is suitably more than 16%, for example 20 to 40%. Suitably the hydroxypropylcellulose has a dynamic viscosity of from 100 to 600cP (for example from 150 to 450cP), wherein the wherein the viscosity is measured in a 2%w/v aqueous solution of the hydroxypropylcellulose at 25°C. Alternatively the hydroxypropyl cellulose may have a degree of substitution of from approximately 5 to approximately 16% hydroxypropoxy groups). Such hydroxypropyl celluloses are commercially available as "low substituted" hydroxypropyl cellulose. Although low substituted hydroxypropylcellulose is often described as water-insoluble, we have surprisingly found that low substituted hydroxypropylcellulose is sufficiently hydrophilic to prevent precipitation of the Agent from solution, and for the purposes of this invention low-substituted hydroxypropylcellulose is to be considered a water-soluble cellulose ether. When the cellulose ether is hydroxyethylcellulose it is suitably a water-soluble hydroxyethylcellulose with a molecular weight of for example, 150,000 to 350,000 such as approximately 220,000 to 270,000. Generally, suitable water-soluble hydroxyethylcelluloses include those with a dynamic viscosity of 50 to 250cP, such as 80 to 125cP, wherein the viscosity is measured in a 2%w/v aqueous solution of the hydroxyethylcellulose at 25°C. Suitably the hydroxyethylcellulose has a degree of substitution of approximately 0.8 to 2.5, such as from 0.8 to 1.5, for example approximately 1, wherein here degree of substitution refers to the average number of hydroxyethyl groups per anyhydroglucose ring of the cellulose.

In a further embodiment the water-soluble cellulose ether is methylcellulose, particularly a methylcellulose with a low viscosity, for example a dynamic viscosity of from 5 to 100cP, such as 10 to 25cP (25w/v solution in water at 20°C measured using for example an Ubbelohde viscometer in accordance with ASTM D2363). Suitably the methylcellulose has a degree of substitution of from 1 to 2, for example from 1.64 to 1.92, such as approximately 1.8, wherein here degree of substitution refers to the average number of methoxy groups per anyhydroglucose ring of the cellulose. Suitably the methylcellulose has a molecular weight of approximately 10,000 to 50,000, for example 10,000 to 35,000. Suitable methylcelluoses are commercially available, for example under the trade name Methocel^{™} such as Methocel^{™} A and Methocel^{™} MC ex Dow Inc.

In another embodiment of the present invention the water-soluble cellulose ether is a water-soluble alkaline metal salt of carboxymethylcellulose, particularly sodium carboxymethylcellulose. Suitably the water-soluble alkaline metal salt of carboxymethylcellulose is one with an average degree of substitution of approximately 0.7 to 1.2, for example from 0.8 to 0.95 wherein here degree of substitution refers to the average number of carboxymethyl groups per anyhydroglucose ring of the cellulose. Generally the water-soluble alkaline metal salt of carboxymethylcellulose is a low viscosity grade, suitably one with a dynamic viscosity of approximately 10 to 2500cP, for example from 10 to 1500cP, such as from 10-15cP or from 900 to 1500cP, wherein the viscosity is measured in a 1%w/v aqueous solution of the carboxymethylcellulose at 25°C.

By "ester of a water-soluble cellulose ether" used herein is meant an ester formed between one or more hydroxyl group(s) present in a water-soluble cellulose ether and one or more suitable organic acid(s) or reactive derivatives thereof, to thereby form ester linked groups on the water-soluble cellulose ether. Suitable water-soluble cellulose ethers are as hereinbefore defined. Suitable organic acids include organic carboxylic acids which may aliphatic or aromatic carboxylic acids. Suitable aliphatic carboxylic acids may be acyclic (branched or unbranched) or cyclic and may saturated or unsaturated.

Particular acyclic aliphatic carboxylic acids include those containing from 2 to 8 carbon atoms, such as from 2 to 5 carbon atoms. Examples of suitable saturated acyclic aliphatic carboxylic acids include acetic acid, propionic acid, butyric acid or valeric acid. The acyclic aliphatic acid may be optionally substituted by one or more groups (for example 1, 2 or 3), which may be the same or different, selected from carboxy and hydroxy. Suitable substituted acyclic aliphatic carboxylic acids include for example acyclic aliphatic dicarboxylic acids such as malonic, succinic, glutaric, fumaric or maleic acid; acyclic aliphatic hydroxy substituted carboxylic acids, for example glycolic or lactic acid; and acyclic aliphatic hydroxy substituted di-or tri-carboxylic acids, for example tartronic, malic, tartaric or citric acid.

Suitable aromatic carboxylic acids include aryl carboxylic acids containing up to 14 carbon atoms. Suitable aryl carboxylic acids comprise an aryl group, for example a phenyl or naphthyl group which carries one or more carboxyl groups (for example 1, 2 or 3 carboxy groups). The aryl group is optionally substituted by one or more groups (for example 1, 2 or 3), which may be the same or different selected from hydroxy, (1-4C)alkoxy (for example methoxy) and sulphonyl. Suitable examples of aryl carboxylic acids include, phthalic, isophthalic, terephthalic or trimellitic acid (1,2,4-benzenetricarboxylic acid).

When the organic acid carries more than one carboxylic acid group, suitably only one carboxy group of the acid forms an ester linkage to the water-soluble cellulose ether. For example in the case of HPMC succinate one carboxy group of each succinate group forms an ester linkage with the cellulose and the other carboxy group is present as the free acid. When the ester of a water-soluble cellulose ether contains free acid groups, such as carboxyl groups, the ester may be used in the free acid form or as a pharmaceutically acceptable salt thereof, for example a sodium salt.

As will be clear, the ester linkages within a water-soluble cellulose ether may be formed by reaction of cellulose or a cellulose ether with a suitable organic acid, as hereinabove described or by a reactive derivative of an organic acid. Suitable reactive derivatives include, for example an acid anhydride such as phthalic anhydride.

The ester of the water-soluble cellulose may carry the same ester linked moieties, for example acetate groups as in the case of HPMC acetate. Alternatively the ester of a water-soluble cellulose ether may carry a plurality of different ester-linked moieties (for example 2 or more moieties such as, for example, succinate and phthalate groups). For example HPMC acetate succinate refers to a mixed ester of HPMC which carries both succinate and acetate groups and HPMC acetate succinate trimellitate is a mixed ester of HPMC which carries acetate, succinate and trimellitate groups.

In a particular embodiment of the present invention the ester of a water soluble cellulose ether is an ester of HPMC or hydroxypropylcellulose (HPC) which carries one or more ester groups selected from acetate, succinate, phthalate, isophthalate, terephthalate and trimellitate. Particular examples of esters of water-soluble cellulose ethers include, but is not limited to, HPMC acetate, HMPC succinate, HPMC acetate succinate, HPMC phthalate (commercially available as, for example HP-55 and HP 55-S), HPMC trimellitate, HPMC acetate phthalate, HPMC Acetate trimellitate, HPC acetate phthalate, HPC butyrate phthalate, HPC acetate phthalate, succinate and HPC acetate trimellitate succinate. More particularly the ester of water-soluble cellulose ether is selected from HPMC acetate succinate (commercially available as Aqoat ex Shin-Etsu Chemical Co. for example Aqoat AS-LG).

In another embodiment of the invention, the water-soluble cellulose ether or ester of a water-soluble cellulose ether is selected from hydroxypropylcellulose, HPMC, hydroxyethylcellulose, methylcellulose, a water-soluble alkaline metal salt of carboxymethylcellulose and an ester of HPMC or hydroxypropyl cellulose which carries one or more ester groups selected from acetate, succinate, phthalate, isophthalate, terephthalate and trimellitate. In this embodiment particular water-soluble cellulose ethers and/or esters of a water-soluble cellulose ethers are selected from hydroxypropyl cellulose, hydroxyethylcellulose, methylcellulose, sodium carboxymethylcellulose and HPMC acetate succinate. More particularly the pharmaceutical composition according to the present invention comprises the Agent and a water-soluble cellulose ether selected from hydroxypropyl cellulose, hydroxyethylcellulose, methylcellulose and sodium carboxymethylcellulose. A particular water-soluble cellulose ether is hydroxypropylcellulose, more particularly a water-soluble hydroxypropylcellulose having a degree of substitution of more than 16%. Another particularly suitable water-soluble cellulose ether in this embodiment is methylcellulose. Suitable grades of the above mentioned water-soluble cellulose ethers are as hereinbefore described.

The composition according to the present invention may contain a single water-soluble cellulose ether or ester of a water-soluble cellulose ether or two or more such compounds.

The water-soluble cellulose ether, or ester of water-soluble cellulose ether is present in the composition in a quantity sufficient to inhibit the rate of precipitation of the Agent from an acidic aqueous solution when the pH of said solution is increased. In particular the water-soluble cellulose ether, or ester of water-soluble cellulose ether is present in the composition in a quantity sufficient to inhibit the rate of precipitation of the Agent from solution in-vivo as the Agent passes from the acidic environment of the stomach to the higher pH regions of the GI tract from which the Agent is thought to be absorbed such as the upper intestine. Suitably, the weight ratio of the Agent to water-soluble cellulose ether, or ester of water-soluble cellulose ether is from 50:1 to 1:5, for example from 35:1 to 1:1, more particularly from 40:1 to 2:1, still more particularly from 33:1 to 2:I such as from 33:1 to 10:1. In a further embodiment the weight ratio of Agent to cellulose ether or ester thereof is up to 32:1, for example from 32:1 to 1:1, more particularly from 30:1 to 2:1, still more particularly from 25:1 to 3:1.

In another embodiment of the invention the weight ratio of Agent to water-soluble cellulose ether, or ester of water-soluble cellulose ether is from 40:1 to 2.5:1, particularly from 30:1 to 3:1 and especially from 5:1 to 3:1. The inventors have found that, in general, increasing the amount of cellulose ether or ester thereof such that the weight ratio of Agent to cellulose ether or ester thereof is less than about 3:1 (for example 1:1) does not result in any significant further reduction in the rate of precipitation of the Agent, beyond that observed when the weight ratio is approximately 3:1.

Suitably the composition contains for example, from 0.05 to 85%, particularly from 0.5 to 50%, more particularly from 1 to 30%, especially from 0.5 to 20% and more especially from 1 to 10% by weight, based upon the total weight of the composition of water-soluble cellulose ethers and esters of water-soluble cellulose ethers.

The composition may contain from 0.01mg to 1g of Agent. Suitably the composition contains a daily dose of the Agent in a quantity sufficient to provide the desired therapeutic benefit. Suitable quantities of the Agent include, for example 10, 15, 25, 50, 75, 100, 125, 150, 200, 250, 300, 350, 400, 450, 500 or 550mg, depending upon the dose required and the particular form of the pharmaceutical composition. In an embodiment the composition contains 100, 150, 250 or 500mg of the Agent, especially 250mg of the Agent.

The Agent may be used in the free base form or as a pharmaceutically acceptable salt, such as a pharmaceutically acceptable mono- or di-acid addition salt with, for example, an inorganic or organic acid, for example hydrochloric acid. In one embodiment, the Agent is in the free base form, particularly a crystalline free-base form. As will be clear, the term "free-base form" refers to the case when the Agent is not in the form of a salt.

Typically the Agent will be present in an amount within the range of from 1 to 99%, and suitably from 1 to 70%, for example from 5 to 65% and especially from 10 to 60% by weight of the composition.

In another embodiment of the present invention the composition further comprises a wetting agent.

In another embodiment of the present invention the composition comprises:
(a) the Agent;
(b) a wetting agent; and
(c) a water-soluble cellulose ether.

We have found that a combination of a wetting agent and a water-soluble cellulose ether, or ester of a water-soluble cellulose ether, in the composition further reduces the rate of precipitation of the Agent from solution at pH values similar to that found in the regions of the GI tract, from which the Agent is thought to be absorbed, such as the upper intestine.

Suitable wetting agents include pharmaceutically acceptable surface-active materials, for example pharmaceutically acceptable surfactants, which may be ionic or non-ionic.

Suitable pharmaceutically acceptable non-ionic surfactants include, for example, polyethylene glycols, polyoxyethylene esters and ethers, for example polyethoxylated castor oil (for example Cremophor EL), polyethoxylated hydrogenated castor oil, polyethoxylated fatty acids derived from castor oil, or polyethoxylated fatty acids derived from hydrogenated castor oil; ethoxylated stearic acid, for example Solutol HS15; and ethylene oxide-propylene oxide copolymers, for example ethylene oxide-propylene oxide block copolymers, such as Pluronic or Tetronic surfactants.

Suitable pharmaceutically acceptable ionic surfactants may be anionic, cationic or zwitterionic. Suitable anionic surfactants include:
(i) fatty acid salts such as alkyl and aryl sulphonates, sulphates or carboxylates, such as an alkali metal (8-22C)alkyl sulphate, sulphonate or carboxylate, particularly an alkali metal (8-20C)alkyl sulphate, sulphonate or carboxylate, for example, sodium dodecyl sulphate (sodium lauryl sulphate), potassium myristate, sodium laurate or sodium lauryl sulphonate;
(ii) di-alkyl sulphoscuccinates, particularly di-(4-12C)alkyl sulphoscuccinates, particularly in the form of an alkali metal or an alkaline earth metal salt such as a sodium, potassium or calcium salt, especially a sodium salt. Particular examples include, sodium, calcium or potassium dioctyl sulphosuccinate (e.g. Docusate sodium or Aerosol OT) or sodium diamyl sulphosuccinate (Aerosol AY);
(iii) bile acid salts, such as salts of deoxycholic acid, taurocholic acid, or glycocholic acid, particularly alkali metal salts of bile acids, for example a sodium salt of a bile acid such as sodium taurocholate, sodium deoxycholate or sodium glycocholate; and
(iv) anionic water-dispersible cellulose derivatives, such as anionic water-dispersible cellulose ethers, for example carboxymethyl cellulose and salts thereof

Suitable cationic surfactants include for example quaternary ammonium compounds such as:
(i) alkylammonium compounds (for example (8-22C)alkylammonium, particularly (8-20C)alkylammonium compounds, such as halides) including, for example, laurylammonium chloride;
(ii) alkyltrimethyl ammonium compounds (for example (8-22C) alkyltrimethyl ammonium, particularly (8-20C)alkyltrimethyl ammonium compounds, such as halides) for example cetyltrimethylammonium bromide (Cetramide), trimethyltetradecylammonium bromide (Myristamide) or lauryl trimethylammonium bromide (Lauramide);
(iii) benzalkonium halides (such as (8-20C)alkylbenzyldimethylammonium halides, particularly (8-18C)alkylbenzyldimethylammonium halides and mixtures thereof), for example benzalkonium chloride; and
(iv) alkylpyridinium compounds such as (8-20C)alkylpyridinium compounds, for example cetylpyridinium chloride or bromide. The composition may contain a single wetting agent or two or more wetting agents.

In an embodiment the wetting agent is a pharmaceutically acceptable cationic or anionic surfactant, for example a quaternary ammonium compound or an alkali metal (8-22C)alkyl sulphate. The inventors have found that the presence of a cationic or anionic pharmaceutically acceptable surfactant (particularly a cationic surfactant) has a surprising synergistic effect upon reducing the rate of precipitation of the Agent compared to the use of the water-soluble cellulose ether or ester thereof alone or the surfactant alone.

In a further embodiment the wetting agent is a cationic surfactant, particularly a quaternary ammonium compound and more particularly a (8-18C)alkylbenzyldimethylammonium halide, for example benzalkonium chloride.

In another embodiment the wetting agent is selected from an anionic and non-ionic surfactant, or a combination thereof. Suitably in this embodiment the wetting agent is an anionic surfactant, particularly an alkali metal (8-20C)alkyl sulphate, more particularly an alkali metal dodecyl sulphate. Still more particularly the wetting agent in this embodiment is sodium dodecyl sulphate (sodium lauryl sulphate).

The wetting agent may be present in the composition according to the present invention at a concentration below the critical micelle concentration (CMC) of the wetting agent. The inventors have found that the wetting agent inhibits precipitation of the Agent from solution at pH values similar to those in the GI tract where the Agent is absorbed (such as the upper intestine). This is surprising because the wetting agent is at a concentration below the CMC required for solubilisation of the Agent by micelle formation.

The CMC for a particular wetting agent in an aqueous environment may be readily determined using standard techniques, for example using the Wilhelmy plate method (see for example S.A Hagan, A.G.A Coombes, M.C. Garnett, S.E. Dunn, M.C. Davies, L. Illum and S.S. Davis, Langmuir 1996, 12, 2153-2161).

A suitable weight ratio of Agent to wetting agent is from 1:2 to 500:1, particularly from 1:1 to 300:1, more particularly from 100:1 to 250:1 and still more particularly from 150:1 to 200:1.

Suitably the composition will contain from 0.01 to 10%, for example from 0.05 to 5%, particularly from 0.1 to 1% and more particularly from 0.1 to 0.5% by weight of the wetting agent.

Suitable water-soluble cellulose ethers and esters of water-soluble cellulose ethers and quantities thereof for use in the compositions described above containing a wetting agent are as hereinbefore defined in relation to the first aspect of the invention.

In view of the foregoing, a particular composition according to this embodiment of the invention comprises:
(a) from 1 to 99 (particularly from 10 to 98) parts the Agent;
(b) from 0.01 to 10 (particularly from 0.05 to 5) parts of a wetting agent (particularly an anionic surfactant, for example sodium dodecyl sulphate); and
(c) from 0.1 to 90 (particularly from 0.5 to 85) parts of a water-soluble cellulose ether (particularly hydroxypropyl methylcellulose);
wherein all parts are by weight and the sum of the parts (a)+(b)+(c)=100.

Another composition according to the present invention comprises:
(a) from 60 to 99 (particularly from 70 to 85) parts the Agent;
(b) from 0 to 10 (particularly from 0.05 to 5) parts of a wetting agent (particularly an anionic surfactant (for example selected from an alkali metal (8-20C)alkyl sulphate, particularly sodium dodecyl sulphate) or a cationic surfactant (for example a quaternary ammonium compound such as a (8-18C)alkylbenzyldimethylammonium halide, for example benzalkonium chloride)); and
(c) from 0.1 to 40 (particularly from 0.5 to 30) parts of a water-soluble cellulose ether selected from hydroxypropyl methylcellulose, hydroxypropyl cellulose, hydroxyethylcellulose, methylcellulose and sodium carboxymethylcellulose (particularly selected from hydroxypropylcellulose, hydroxyethylcellulose, methylcellulose and sodium carboxymethylcellulose and especially a water-soluble hydroxypropyl cellulose);
wherein all parts are by weight and the sum of the parts (a)+(b)+(c)=100; and
when the water-soluble cellulose ether is HPMC the weight ratio of Agent to HPMC is 32:1 or
less (for example from 32:1 to 1:1 such as from 30:1 to 2:1).

The water-soluble cellulose ether and/or ester of a water-soluble cellulose ether present in the composition according to the invention may be used as, for example fillers, binders, disintegrants or film coatings as, for example, described hereinafter.

Optionally additional excipients may be included in the pharmaceutical composition according to the present invention. Additional excipients which may be present include for example, one or more fillers (diluents), binders, disintegrants or lubricants.

Accordingly a further embodiment of the invention provides a pharmaceutical composition comprising the Agent, a water-soluble cellulose ether and/or ester of a water-soluble cellulose ether, a wetting agent and one or more fillers, binders, disintegrants or lubricants. A still further embodiment of the invention provides a solid pharmaceutical composition for oral administration comprising the Agent, a water-soluble cellulose ether and/or ester of a water-soluble cellulose ether, a wetting agent one or more fillers, one or more binders, one or more disintegrants and one or more lubricants.

Suitable fillers include, for example, lactose (which may be in an anhydrous or hydrated form, for example lactose monohydrate), sugar, starches (for example corn, wheat, maize, potato), modified starches (for example as starch hydrolysates or pregelatinized starch which may be thermally, mechanically or chemically modified), microcrystalline starches, mannitol, sorbitol, trehalose, maltose, inorganic salts (e.g. calcium carbonate, magnesium carbonate, dibasic calcium phosphate (anhydrous/dihydrate), tribasic calcium phosphate), cellulose, cellulose derivatives (e.g. microcrystalline cellulose), calcium sulphate, xylitol and lactitol.

Suitable binders include, for example, polyvinylpyrrolidone (for example povidone K25-32, particularly K29-32, wherein the "K value" is an indication of the average molecular weight range obtained from the Fikentscher equation described in the Handbook of Pharmaceutical Excipients, 3^{rd} Edition 2000 American Pharmaceutical Association p 433), lactose (which may be in an anhydrous or hydrated form, for example lactose monohydrate), starches, modified starches, sugars, gum acacia, gum tragacanth, guar gum, pectin, wax binders, microcrystalline cellulose, methylcellulose, carboxymethylcellulose and salts thereof (for example sodium carboxymethylcellulose), hydroxypropyl methylcellulose, hydroxyethyl cellulose, hydroxypropyl cellulose, copolyvidone, gelatin and alginates (for example sodium alginate).

Suitable disintegrants include, for example, croscarmellose sodium, crospovidone, polyvinylpyrrolidone, sodium starch glycolate, starches, microcrystalline cellulose carboxymethylcellulose and salts thereof (for example sodium or calcium carboxymethylcellulose), hydroxypropyl methylcellulose, hydroxypropylcellulose (particularly low substituted hydroxypropylcellulose i.e. hydroxypropylcellulose containing approximately 5 to 16% by weight hydroxypropoxy groups) or alginic acid.

Suitable lubricants include, for example, magnesium stearate, stearic acid, palmitic acid, calcium stearate, talc, carnauba wax, hydrogenated vegetable oils, mineral oil, polyethylene glycols, sodium lauryl sulphate and sodium stearyl fumarate.

Further additional excipients which may be added include preservatives, stabilisers, anti-oxidants, silica flow conditioners, antiadherents or glidants.

Other suitable fillers, binders, disintegrants, lubricants and additional excipients which may be used are described in Handbook of Pharmaceutical Excipients, 3^{rd} Edition (2000), American Pharmaceutical Association; The Theory and Practice of Industrial Pharmacy, 3^{rd} Edition, Lachman et al. 1986; Pharmaceutical Dosage Forms: Tablets Volume 1, 2^{nd} Edition, Lieberman, Hebert A., et al, 1989; Modem Pharmaceutics, Banker, Gilbert and Rhodes, Christopher T, 3^{rd} edition, 1995; and Remington's Pharmaceutical Sciences, 20^{th} Edition, 2000.

Suitably one or more fillers will be present in an amount of from 10 to 90% by weight, for example from 30 to 50% by weight.

Suitably one or more binders will be present in an amount of from 0.5 to 50% by weight, for example from 0.5 to 10% by weight.

Suitably one or more disintegrants will be present in an amount of from 0.5 to 20%, for example from 1 to 10% by weight.

Suitably one or more lubricants will be present in an amount of from 0.1 to 5% by weight, for example from 0.5 to 3% by weight.

It will be appreciated that a particular excipient may act as a both a binder and a filler, or as a binder, filler and disintegrant. Typically the combined amount of filler, binder and disintegrant comprises, for example 40 to 80% by weight of the composition.

In a further embodiment the composition according to the present invention comprises:
(a) from 10 to 80 parts of the Agent;
(b) from 0.05 to 5 parts wetting agent selected from an anionic surfactant (particularly sodium dodecyl sulphate);
(c) from 10 to 60 parts of one or more fillers selected from lactose (particularly lactose monohydrate), mannitol and microcrystalline cellulose;
(d) from 1 to 10 parts of one or more disintegrants selected from carboxymethylcellulose sodium, carboxymethyl cellulose calcium, croscarmellose sodium, crospovidone and sodium starch glycolate ;
(e) from 1 to 20 parts of a binder selected from a polyvinylpyrrolidone (particularly povidone (more particularly K29-32) and hydroxypropyl methylcellulose (particularly grades, 1828, 2208, 2906 and more particularly 2910 having a dynamic viscosity of from 2 to 18 cP as described hereinbefore); and
(f) 0 to 3 parts of a lubricant (such as magnesium stearate);
wherein all parts are by weight and the sum of the parts (a)+(b)+(c)+(d)+(e)+(f)=100, and wherein at least one of the components selected from (d) or (e) contains a water-soluble cellulose ether selected from hydroxypropyl methylcellulose and carboxymethylcellulose sodium.

In this embodiment it is preferred that component (e) of the composition contains hydroxypropyl methylcellulose.

The pharmaceutical composition according to the present invention is suitably prepared as a physical mixture comprising the Agent and a water-soluble cellulose ether or ester of a water-soluble cellulose ether. Alternatively the Agent and water-soluble cellulose ether or ester of a water-soluble cellulose ether may be administered to a patient separately or simultaneously. When the Agent and water-soluble cellulose ether or ester of a water-soluble cellulose ether are administered separately the components of the composition may be administered in any order, for example the Agent followed by the cellulose or vice versa. When separate administration is used the interval between the administration of the two components of the composition should be such that the inhibitory effect on precipitation of the Agent is not lost. Typically administration within less than 20, for example less than 10 minutes should be sufficient. Generally, however, the components of the present composition are administered substantially simultaneously, conveniently as a single formulation containing all of the components of the composition such as a tablet or capsule formulation.

In a preferred embodiment the pharmaceutical composition of the invention is formulated into an oral dosage form, for example as a powder mixture or a liquid formulation such as a solution or suspension in a suitable liquid medium. Generally, however, the pharmaceutical composition is prepared as a solid dosage form suitable for oral administration, particularly a solid unit dosage form suitable for daily oral administration. Examples of suitable solid dosage forms include tablet, pellet, granule or capsule formulations.

When the pharmaceutical composition according to the invention is a solid dosage form such as a tablet, pellet or granules the solid composition optionally further comprises a suitable coating, for example a film coating. A coating can be used to provide protection against, for example, moisture ingress or degradation by light, to colour the formulation, or to modify or control the release of the Agent from the formulation.

Suitable coatings, such as film coatings, that may be applied to the composition according to the invention comprise a film-forming agent, for example a sugar or more particularly a film-forming polymer. Suitable sugar coatings are well known and comprise for example sucrose or lactose. Suitable film-forming agents include, for example film-forming polymers, such as cellulose ethers, esters and mixed ethers and esters, including esters of water-soluble cellulose ethers, for example hydroxypropyl methylcellulose, hydroxypropyl ethylcellulose, hydroxypropylcellulose, methylcellulose, hydroxypropyl methylcellulose acetate succinate or hydroxypropyl methylcellulose phthalate; film-forming acrylic polymers, for example methacrylate-methylmethacrylate copolymers; and film-forming vinyl polymers, for example polyvinyl alcohols or polyvinyl acetate phthalate. Suitably the film-forming polymer is a water-soluble film-forming polymer, particularly a water-soluble cellulose ether for example hydroxypropyl methylcellulose (particularly hydroxypropyl methylcellulose with a dynamic viscosity of from 2 to 18cP (measured in a 2%w/v solution at 20°C) and selected from, for example grades 1828, 2208, 2906 and especially 2910 as defined hereinbefore). The amount of film-forming agent used will depend upon the desired properties of the film coating. Generally the film forming agent will be present in an amount of from 40 to 90% by weight of the film coating, for example from 50 to 80% of the film coating. The film-forming agent is typically present at from 0.5 to 5%, suitably from 1 to 3% by weight of the formulation according to the invention.

Optionally the film coating contains additional components such as plasticiser, colorants, dispersion aids and opacifiers. Plasticisers may be used to improve film flexibility and durability and adhesion properties of the film coating. Suitable plasticisers include, for example glycerin, acetylated monoglycerides, citrate esters (for example triethyl citrate), propylene glycols, polyethylene glycols (for example polyethylene glycols with a molecular weight of from 200 to 500, particularly 300), triacetin (glycerol tri-acetate), triglycerides (for example castor oil), or phthalate esters (for example diethylphthalate). Generally the plasticiser, when used, is present in an amount of from 1 to 20%, for example 5 to 15% by weight based upon the weight of the film coating.

Suitable opacifiers and colorants are well known and include for example titanium dioxide, ferric oxides (for example iron oxide).

Suitable dispersion aids include, for example talc.

In an embodiment of the invention the film coating comprises
(i) from 50 to 100 (suitably from 50 to 80 parts of a water-soluble cellulose ether (suitably hydroxypropyl methylcellulose, particularly hydroxypropyl methylcellulose with a dynamic viscosity of from 2 to 18cP (measured in a 2%w/v solution at 20°C), for example grades 2910, 1828, 2208 or 2906 as defined hereinbefore with a dynamic viscosity of from 5 to 7cP);
(ii) from 0 to 25 (particularly from 5 to 20) parts plasticiser (suitably polyethylene glycol, particularly polyethylene glycol with a molecular weight of from 200 to 500); and
(iii) from 0 to 50 (particularly from 0 to 30) parts in total of opacifiers (suitably titanium dioxide), colorants (suitably an iron oxide) and dispersion aids;
wherein all parts are by weight and the sum of the parts (i)+(ii)+(iii) = 100.

The coating may comprise, for example, 0.5 to 10% by weight of the composition, particularly 1 to 6%, and preferably 2 to 3%. Suitable film coatings are commercially available as concentrates that may be diluted with water and optionally a cellulose ether such as HPMC and a plasticiser such as polyethylene glycol prior to application to the composition. Such concentrates include Opaspray^{™} coatings ex Colorcon, for example Opaspray^{™} Brown M-1-25092 and Opaspray Yellow M-1-22842.

In a particular embodiment of the invention the pharmaceutical composition comprises a solid pharmaceutical composition (such as a tablet, pellet or granule formulation) comprising:
(i) a core comprising the Agent; and
(ii) a coating comprising an ester of a water-soluble cellulose ether or (preferably) a water-soluble cellulose ether.

In this embodiment suitable water-soluble cellulose ethers are as hereinbefore described, especially hydroxypropyl methylcellulose (particularly grades, 1828, 2208, 2906 and especially 2910 having a dynamic viscosity of from 2 to 18 cP). Suitable esters of water-soluble ethers for use in this embodiment are as hereinbefore defined in relation to the first aspect of the present invention. Suitably the coating is applied as a film coating as herein described. The core comprising the Agent may comprise any of the compositions described hereinbefore containing a water-soluble cellulose ether or ester of a water-soluble cellulose ether (and optionally other additional excipients as hereinbefore described). Alternatively, the core may comprise the Agent without a water-soluble cellulose ether or ester of a water-soluble cellulose ether. Accordingly, in this embodiment the water-soluble cellulose ether, or ester of a water-soluble cellulose ether may be present entirely in the coating. Alternatively, water-soluble cellulose ether(s) or ester of a water-soluble cellulose ether may be present in both the core and the coating.

In a further embodiment of the present invention there is provided a solid pharmaceutical composition (such as a tablet, pellet or granule formulation) comprising (i) a core comprising the Agent; and (ii) a coating (particularly a film coating), wherein a water-soluble cellulose ether and/or an ester of a water-soluble cellulose ether is present in at least one of the core or coating. In this embodiment the water-soluble cellulose ether, or ester of a water-soluble cellulose ether may be present entirely in the coating, entirely in the core or be present in both the core and the coating.

In another particular embodiment the composition according to the present invention is a tablet, pellet or granule suitable for oral administration, comprising a core coated with a film coating wherein the core comprises:
from 45 to 55% of the Agent (particularly in free-base form);
from 25 to 40% lactose (particularly lactose monohydrate);
from 5 to 15% microcrystalline cellulose;
from 2 to 6% disintegrant (particularly croscarmellose sodium);
from 1 to 5% povidone (particularly K29-32);
from 0.05 tol% (particularly 0.1 to 0.5%) sodium dodecyl sulphate; and
from 0.1 to 4% lubricant (particularly magnesium stearate);
and wherein the film coating comprises:
from 0.5 to 3% water-soluble cellulose ether (particularly hydroxypropyl
methylcellulose, more particularly grade 2910, with a dynamic viscosity of from 5 to 7cP);
from 0 to 0.5% (particularly from 0.05 to 0.5%) plasticiser (particularly polyethylene glycol, more particularly polyethylene glycol with a molecular weight of 200 to 500, especially 300);
from 0 to 0.5% (particularly 0.1 to 0.4%) dispersion aid (suitably talc);
from 0 to 0.5% (particularly 0.1 to 0.4%) opacifier (suitably titanium dioxide); and
from 0 to 0.5%, (particularly from 0.001 to 0.4%) colorant (particularly iron oxide);
wherein all % are by weight based upon the total weight of the composition.

The pharmaceutical composition of the invention may be prepared, using standard techniques and manufacturing processes generally known in the art, for example by dry blending the components or, particularly, wet granulation techniques followed by compression to form a tablet or filling into suitable capsules. A suitable wet granulation technique comprises for example, blending together the Agent, one or more fillers, all or a portion of a disintegrant and optionally the water-soluble cellulose ether and/or ester of a water-soluble cellulose ether, wetting agent and/or one or more binders, as well as other additional excipients if desired, using, for example a granulator. The resulting powder blend is then granulated with a small volume of purified water, optionally containing wetting agent and/or one or more binders (which may be a water-soluble cellulose ether). The granulate is passed through a screen, to break up large aggregates, dried and passed though a mill. Any remaining disintegrant and a lubricant are then added to the milled granulation and after blending the resultant homogeneous mixture is compressed into tablets. Alternatively, the milled granulate is filled into a suitable capsule to provide a capsule formulation.

A suitable dry blending technique comprises for example, blending together the Agent, the water-soluble cellulose ether and/or ester of a water-soluble cellulose ether and optionally wetting agent, one or more fillers, one or more binders and one or more disintegrants, as well as other additional excipients if desired. The components of the blend prior to blending, or the blend itself, may be passed through a mesh screen, for example a 400-700 µm mesh screen. A lubricant, which may also be screened, is then added to the blend and blending continued until a homogeneous mixture is obtained. The mixture is then compressed into tablets. Alternatively, the mixture may be filled into suitable capsules to provide a capsule formulation.

It will be appreciated that modifications of the dry blending and wet granulation techniques, including the order of addition of the components and their screening and blending prior to compression into tablets, may be carried out according to principles well known in the art.

When the composition is coated, for example with a film coating, the coating may then be applied using conventional methods, for example by coating with a film coating formulation, particularly a water-based film coating formulation. The film-coating formulation may be applied to the composition according to the present invention by, for example, spray coating or fluidised bed coating. The provision of a film coating comprising a water-soluble cellulose ether and/or or ester of a water-soluble cellulose ether can be conveniently used to provide the water-soluble cellulose ether or ester of a water-soluble cellulose ether present in the composition according to the present invention.

When the composition is prepared as a capsule formulation the composition is first prepared as a powder or granules and is then filled into a capsule to provide a capsule formulation, suitable capsules are well known in the art. For example, hard gelatin, water-soluble cellulose ether (for example hydroxypropyl methylcellulose) and starch capsules. When the capsule contains a water-soluble cellulose ether, the capsule may be used to provide the water-soluble cellulose ether present in the composition according to the invention.

Thus further aspect of the present invention provides a method of preparing a pharmaceutical composition which comprises admixing the Agent with a water-soluble cellulose ether and/or or ester of a water-soluble cellulose ether and optionally other excipients, wherein the Agent, water-soluble cellulose ether, ester of a water-soluble cellulose ether and optional excipients are as hereinbefore described in relation to the first aspect of the invention. Suitable methods for admixing the components of the composition are as hereinbefore described.

The Agent possesses anti-proliferative activity and accordingly the compositions according to the present invention are useful in the treatment of conditions such as those described in International Patent Application WO 96/33980. For example, the composition of the invention is useful for the treatment of many common human cancers such as lung (including small cell lung cancer and non small cell lung cancer), breast, prostate, ovarian, colorectal, gastric, brain (including glioma and pituitary adenoma), head and neck, bladder, pancreas, oesophageal, stomach, renal, skin (including malignant melanoma), gynaecological (including cervical, endometrial, vaginal, vulval and uterine) and thyroid cancer and in the treatment of a range of leukaemias, lymphoid malignancies and solid tumours such as carcinomas and sarcomas. It is further expected that the compositions of the invention will be useful for the treatment of other diseases involving excessive cellular proliferation such as benign skin hyperplasia, for example psoriasis, and benign prostatic hypertrophy (BPH).

A further aspect of the present invention provides a pharmaceutical composition according to the invention as hereinbefore defined for use as a medicament.

The Agent present in the compositions of the invention possesses anti-proliferative properties such as anti-cancer properties which are believed to arise from its erbB1 receptor tyrosine kinase inhibitory activity. Accordingly the composition of the invention is expected to be useful in the treatment of diseases or medical conditions mediated alone or in part by erbB1 receptor tyrosine kinases, i.e. the composition of the invention may be used to produce an erbB1 receptor tyrosine kinase inhibitory effect in a warm-blooded animal in need of such treatment. Thus the composition of the invention provides a method for treating the proliferation of malignant cells characterised by inhibition of erbB1 receptor tyrosine kinases, i.e. the composition of the invention may he used to produce an anti-proliferative effect mediated alone or in part by the inhibition of erbB1 receptor tyrosine kinase. Accordingly the active substance of the invention is expected to be useful in the treatment of psoriasis and/or cancer by providing an anti-proliferative effect, Particularly in the treatment of erbB1 receptor tyrosine kinase sensitive cancers such as the cancers hereinbefore described.

In an embodiment of the invention there is provided, a pharmaceutical composition according to the invention as hereinbefore defined for use in producing an anti-proliferative effect in a warm-blooded animal (preferably a human). In another embodiment there is provided a pharmaceutical composition according to the invention as hereinbefore defined for use in the treatment of cancer. In a still further embodiment there is provided a pharmaceutical composition according to the invention for use in the prevention or treatment of tumours which are sensitive to the inhibition of erbB 1 receptor tyrosine kinase.

A further aspect of the present invention provides the use of a composition according to the invention as hereinbefore defined in the manufacture of a medicament for use in producing an anti-proliferative effect in a warm-blooded animal (preferably a human).

A further aspect of the present invention provides the use of a composition according to the invention as hereinbefore defined in the manufacture of a medicament for use in use in the treatment of cancer.

The pharmaceutical composition according to the invention may be used in a method for inhibiting the rate of precipitation of The Agent from solution in the G1 tract of a patient in need of the Agent, comprising orally administering to said patient a composition according to the first aspect of the present invention as hereinbefore defined.

A further aspect of the present invention provides the use of a composition according to the first aspect of the invention as hereinbefore defined in the manufacture of a medicament for inhibiting the rate of precipitation of the Agent from solution in the GI tract of a warm blooded mammal (preferably a human).

A further aspect of the present invention provides the use of a water-soluble cellulose ether or an ester or a water-soluble cellulose ether in the manufacture of a medicament containing the Agent for inhibiting the rate of precipitation of the Agent from solution in the GI tract of a warm blooded mammal (preferably a human).

The pharmaceutical composition according to the invention may be used in a method for reducing inter-patient variability in bioavailability and/or plasma concentrations of the Agent in a patient in need of the Agent comprising orally administering to said patient a pharmaceutical composition according to the first aspect of the present invention as hereinbefore defined.

A further aspect of the present invention provides the use of a pharmaceutical composition according to the first aspect of the present invention as hereinbefore defined in the manufacture of a medicament for reducing inter-patient variability in bioavailability and/or plasma concentrations of the Agent.

A further aspect of the present invention provides the use of a water-soluble cellulose ether or an ester or a water-soluble cellulose ether in the manufacture of a medicament containing the Agent for reducing inter-patient variability in bioavailability and/or plasma concentrations of the Agent.

In the above aspects of the invention the Agent may be administered together with the water-soluble cellulose ether or ester of a water-soluble cellulose ether, alternatively the agent and cellulose ether or ester thereof may be administered separately as individual dosage forms containing the Agent and cellulose respectively. Generally, however, the agent and water-soluble cellulose ether or ester of a water-soluble cellulose ether are administered together, for example as a physical mixture, in a suitable oral dosage form such as a tablet, granule or capsule formulation.

According to a further aspect of the invention there is provided a kit comprising a unit dosage form comprising the Agent and a unit dosage form containing a water-soluble cellulose ether or an ester of a water-soluble cellulose ether, optionally with instructions for use of the kit. The unit dosage forms in the kit may be administered to a patient simultaneously or sequentially as hereinbefore described.

A further aspect of the present invention provides the use of a water-soluble cellulose ether or an ester of a water-soluble cellulose ether to inhibit the precipitation of the Agent from an aqueous solution. More particularly, the use according to this aspect of the present invention reduces the rate of precipitation of the Agent from an aqueous solution induced by a change in pH of the solution, particularly an increase in pH, compared to the rate of precipitation of the Agent from the same aqueous solution in the absence of a water-soluble cellulose ether or an ester of a water-soluble cellulose ether. Still more particularly the use according to this aspect of the invention inhibits precipitation of the Agent from an aqueous solution in-vivo as the Agent passes from the stomach of a patient to the higher pH regions of the GI tract from which the Agent is absorbed (the primary site of which is thought to be the upper intestine). As mentioned herein the water-soluble cellulose ether or an ester of a water-soluble cellulose ether is suitably mixed with the agent to provide a pharmaceutical composition as described herein. Accordingly in an embodiment of the invention there is provided the use of a water-soluble cellulose ether or an ester of a water-soluble cellulose ether in a pharmaceutical composition adapted for oral administration (suitably a solid pharmaceutical composition such as a tablet or capsule) to inhibit precipitation of the Agent from aqueous solution in-vivo following administration of the pharmaceutical composition to a patient.

The dose required of the composition of the invention for the therapeutic or prophylactic treatment of a particular proliferative disease will necessarily be varied depending on for example, the host treated and the severity of the illness being treated. Preferably a daily dose of the Agent in the range, for example, 0.5 to 15 mg per kg body weight is received. More preferably a daily dose of the composition containing the Agent in the range, for example, 1 to 10 mg per kg body weight is received. A unit dose of the composition containing the Agent in the range, for example, 1 to 1000 mg, conveniently 100 to 750 mg, more conveniently 200 to 600 mg, preferably about 250 mg is envisaged. Suitable ratios of Agent to water-soluble cellulose ether or ester of a water-soluble cellulose ether in such unit dosage forms areas hereinbefore defined.

The invention is illustrated below by the following non-limiting examples, wherein the Agent is in the free base form of formula I.

In the Examples the following abbreviations have been used:
HPLC: High performance liquid chromatography
ACN: Acetonitrile.
HPMC: Hydroxypropyl methylcellulose.

### Brief Description of Figures

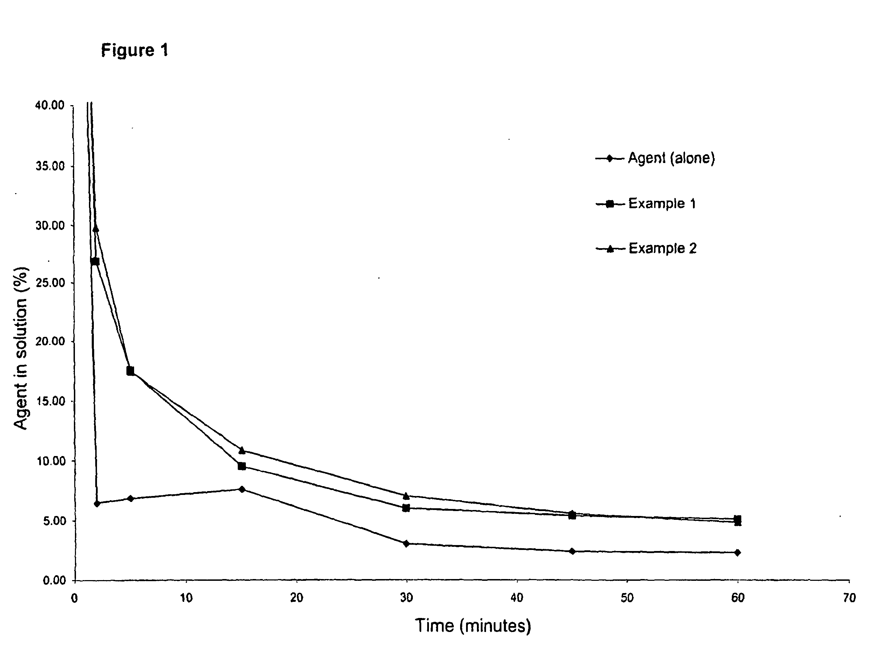

Figure 1 shows the rate of precipitation of the Agent following dissolution of film coated tablet formulations containing the Agent in an acidic medium and a subsequent shift in pH to 6.5. The square data points refer to the film coated tablet formulation described in Example 1, the triangular data points refer to the film coated tablet formulation of Example 2 and the diamond shaped data points show the precipitation of the Agent alone.

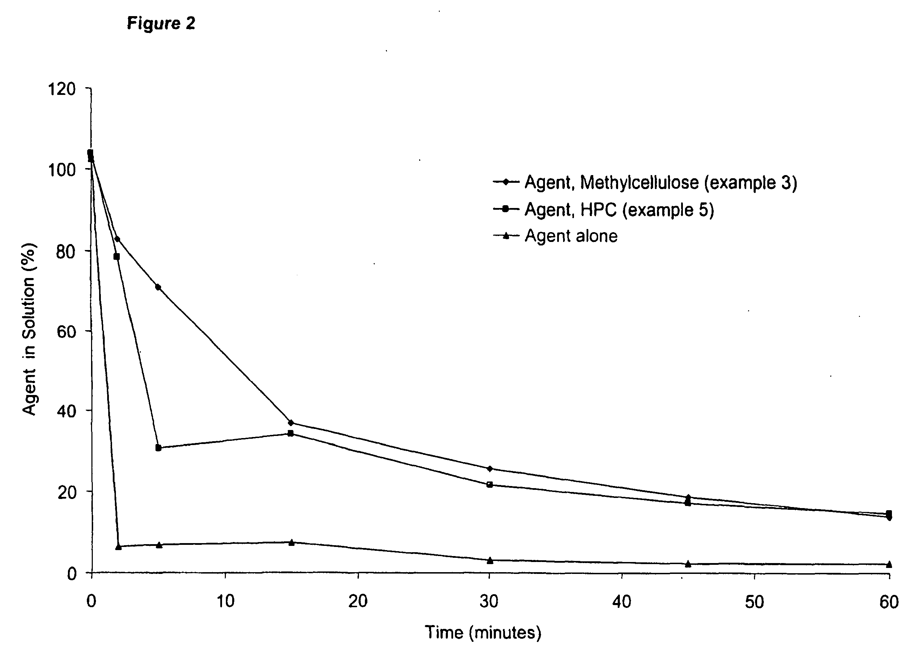

Figure 2 shows the rate of precipitation of Agent from solution following dissolution and a shift in pH of (i) a composition comprising a mixture of the Agent and methylcellulose (Example 3-diamond data points) and (ii) a composition comprising a mixture of the Agent and hydroxypropylcellulose (Example 5 -square data points), compared to precipitation of the Agent alone.

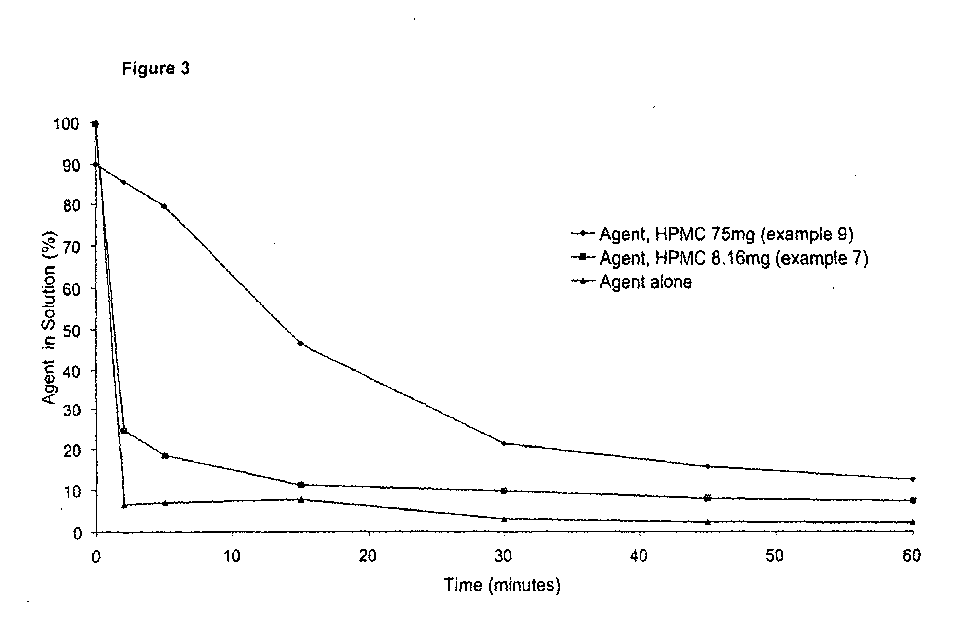

Figure 3 shows the effect of increasing the quantity of HPMC relative to the Agent in a composition on the rate of precipitation of the Agent following dissolution and a shift to pH 6.5. The diamond data points show the composition of Example 9 (75mg HPMC), the square data points show the composition of Example 7 (8.16mg HPMC) and the triangular data points the control sample of the Agent alone. In each case in Figure 3, 250mg of the Agent was used.

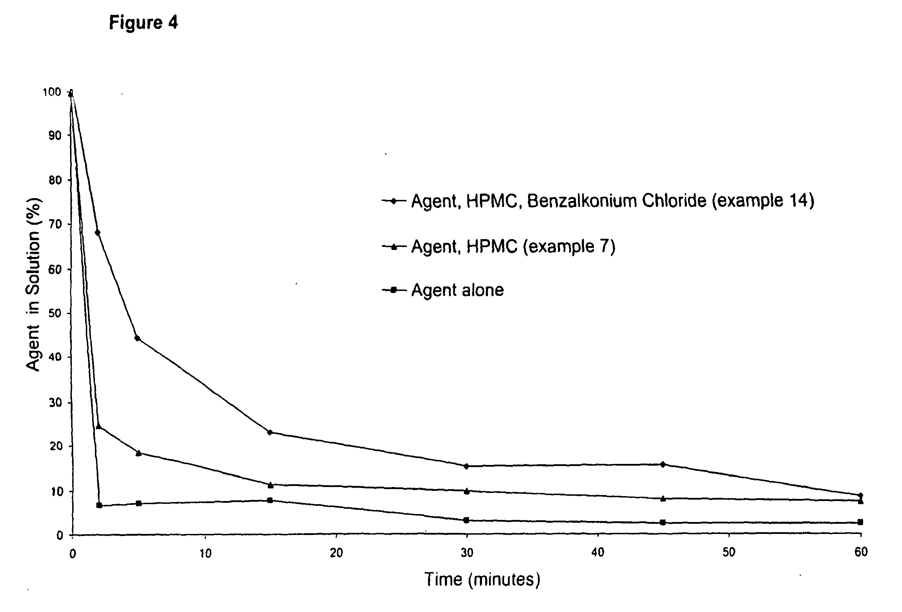

Figure 4 shows the additional effect of a wetting agent together with a water soluble cellulose ether on the rate of precipitation of the Agent following a shift to pH 6.5. The triangular data points show the % Agent in solution following shift of pH of a composition containing the Agent and HPMC (8.16 mg). The diamond shaped data points show the effect of a composition containing the Agent, HPMC (8.16 mg) and benzalkonium chloride (1.5 mg) on the rate of precipitation of the Agent following pH shift. The square data points show the control sample containing the Agent alone. In each case in Figure 4, the compositions contained 250 mg of the Agent.

### Example 1: Coated Tablet Formulation

| **Tablet core** | |
|---|---|
| The Agent | 250.0 mg |
| Lactose monohydrate² | 163.5 mg |
| Microcrystalline cellulose⁴ | 50.0 mg |
| Croscarmellose sodium ⁵ | 20.0 mg |
| Povidone³ | 10.0 mg |
| Sodium lauryl sulphate⁶ | 1.5 mg |
| Magnesium stearate⁷ | 5.0 mg |

| **Tablet coating** | |
|---|---|
| Hydroxypropyl methylcellulose¹ | 8.16 mg |
| Polyethylene glycol 300⁸ | 1.60 mg |
| Talc⁹ | 1.18 mg |
| Titanium Dioxide⁹ | 1.18 mg |
| Yellow ferric oxide⁹ | 0.04 mg |

The example formulation was prepared by conventional wet granulation, compression and film coating processes. The Agent, lactose monohydrate, microcrystalline cellulose and croscarmellose sodium were mixed together in a high shear granulator to produce a homogeneous mix. An aqueous solution of the povidone and sodium lauryl sulphate was then added to the powders with mixing until a suitable wet mass was obtained . The wet granules were passed through a suitable screen to remove large particles, then dried. The dried granules were then passed through a further screen and blended with pre-milled magnesium stearate. The resultant granules were compressed into tablet cores, which were then coated using a conventional pan coater. The film coat was applied by spraying an aqueous suspension of the hydroxypropyl methylcellulose, polyethylene glycol 300, talc, titanium dioxide and yellow ferric oxide onto the tablet cores.

The tablets were tested using the pH shift precipitation method detailed below.

### Example 2 Coated Tablet Formulation

| **Tablet core** | |
|---|---|
| The Agent | 250.0 mg |
| Lactose monohydrate² | 163.5 mg |
| Microcrystalline cellulose⁴ | 50.0 mg |
| Croscarmellose sodium⁵ | 20.0 mg |
| Povidone³ | 10.0 mg |
| Sodium lauryl sulphate⁶ | 1.5 mg |
| Magnesium stearate⁷ | 5.0 mg |

| **Tablet coating** | |
|---|---|
| Hydroxypropyl methylcellulose¹ | 7.65 mg |
| Polyethylene glycol 300⁸ | 1.5 mg |
| Titanium Dioxide¹⁰ | 0.50 mg |
| Yellow ferric oxide¹⁰ | 0.90 mg |
| Red ferric oxide¹⁰ | 0.90 mg |

| | |
|---|---|
| Footnotes: The following excipients were used in Examples 1 and 2: [1] HPMC 606 Grade 2910, 6cP dynamic viscosity (measured at 2%w/v in water at 20°C) ex Shin Etsu). [2] lactose monohydrate - Pharmatose (ex. DMV International, Veghel, The Netherlands), [3] Plasdone^{®} povidone K29-K32 (International Speciality Products, Wayne, New Jersey, USA). [4] Avicel^{®} microcrystalline cellulose (ex. FMC International, Philadelphia, Pennsylvania, USA). [5] Ac-Di.Sol^{®} (FMC International, Philadelphia, Pennsylvania, USA). [6] Sodium Lauryl Sulphate, Albright and Wilson, Oldbury, UK. [7] Magnesium Stearate ex. Mallinckrodt, St Louis, Missouri, USA. [8] Polyethylene glycol 300, Reagent Chemical Services Ltd (Runcorn UK). [9] Yellow iron oxide, titanium dioxide, talc and a portion of the HPMC606 were provided in Opaspray Yellow M-1-22842, ex. Colorcon Ltd, Dartford, Kent, UK. [10] Red and yellow iron oxides, the titanium dioxide and a portion of the HPMC 606 were provided in Opaspray Brown M-1-25092, Colorcon Ltd, Dartford, Kent, UK. | |

The formulation described above was prepared using an analogous wet granulation, compression and film coating method to that described in Example 1. The film coating was applied using a concentrate comprising the red ferric oxide, yellow ferric oxide and titanium dioxide (Opaspray^{™} Brown M-1-25092, ex Colorcon). The concentrate was diluted in a base containing water, polyethylene glycol 300 and HMPC to provide the film coat which was then applied to the tablet cores in a pan coater.

The tablets were tested using the pH shift precipitation method detailed below.

### pH Shift Dissolution Method

The formulations described in the examples above were dissoluted in media comprising 500ml 0.07N HCl (approximately pH 1.5) and sodium chloride (0.2% w/v) for one hour at 37°C (paddle speed 100 rpm). A 5 ml sample was taken at 60 minutes and the media replaced. HPLC analysis (described below) of this sample confirmed that 100% of the Agent was in solution.

10ml of a 2.5M KH₂PO₄ / 16.72% (w/v) NaOH solution was then added to shift the pH to 6.5. 5ml samples were then removed with a plastic syringe at 2, 5, 15, 30, 45 and 60 minutes after pH adjustment and media replaced after every sampling time point. Each sample was centrifuged (14,000rpm) at ambient temperature for 15 minutes and then analysed by HPLC using the following conditions:

| | |
|---|---|
| Eluent: | 38% ACN / 62% water / 0.6% ammonium acetate |
| column: | 10 cm x 3 mm (internal diameter) INERTSIL ODS-3¹¹. (with guard) |
| detection wavelength: | 247 nm |
| flow rate: | 0.9 ml/min |
| injection volume: | 20 µl |
| retention time: | approximately 6 minutes |

| | |
|---|---|
| Footnote [11]: column ex Hichrom containing 3µm beads. | |

### Comparative Example 1

The pH shift precipitation method described above was repeated but using 250mg of the Agent alone.

### Results

Figure 1 shows the pH shift precipitation profiles for the formulations detailed in Examples 1 and 2 and comparative Example 1. The results demonstrate that the rate of precipitation of the Agent following shift of pH to 6.5 is slower for the compositions according to the present invention (Examples 1 and 2) than for the Agent alone, indicating that supersaturation is maintained for longer when the Agent is included in a composition according to the present invention.

The pH shift precipitation test simulates the effect of the change from low pH to high pH as the Agent moves from the acid environment of the stomach into the alkaline environment of the upper intestine which is thought to be the site of highest intrinsic absorption of the Agent. Figure 1 clearly shows that the compositions according to the present invention significantly reduce the rate of precipitation of the Agent from solution following shift to pH 6.5. This would be expected to give an improvement in pharmacokinetic properties for example, increased absorption and/or bioavailability and may reduce inter-patient variability in the bioavailability and/or plasma concentration of the Agent.

### Example 3 to 13

The compositions described below were prepared by weighing the required amount of Agent and excipient(s) into a mortar. The components were then mixed with the pestle until a visually homogenous mix was obtained.

### Example 3

Agent 250mg
Methylcellulose 8.16mg

### Example 4

Agent 250mg
Sodium Carboxymethylcellulose 8.16mg

### Example 5

Agent 250mg
Hydroxypropylcellulose 8.16mg

### Example 6

Agent 250mg
Hydroxyethylcellulose 8.16mg

### Example 7

Agent 250mg
Hydroxypropyl methylcellulose 8.16mg

### Example 8

Agent 250mg
Hydroxypropyl methylcellulose 100mg

### Example 9

Agent 250mg
Hydroxypropyl methylcellulose 75mg

### Example 10

Agent 250mg
Hydroxypropyl methylcellulose 25mg

### Example 11

Agent 250mg
Hydroxypropyl methylcellulose acetate succinate 8.16mg

### Example 12

Agent 250mg
Hydroxypropyl methylcellulose phthalate 8.16mg

### Example 13

Agent 250mg
Hydroxypropyl methylcellulose 8.16mg
Sodium Lauryl Sulphate 1.5mg

### Example 14

A composition containing:
Agent 250mg;
Hydroxypropyl methylcellulose 8.16mg; and
Benzalkonium chloride 1.5mg
was prepared in-situ in the acidic medium of the pH shift dissolution test (as described above in relation to Examples 1 and 2) by preparing a composition containing the Agent (250mg) and hydroxypropyl methylcellulose (8.16mg) using the method described for Example 7 above. This composition was then added to the acidic dissolution medium of the pH dissolution test and, for convenience, benzalkonium chloride was added to the dissolution medium containing the Agent, hydroxypropyl methylcellulose. The composition described above may also be prepared by adding the benzalkonium chloride directly to the mixture of the Agent and hydroxypropyl methylcellulose. This method is particularly suitable for the preparation of larger batches of the composition.

### Comparative Examples 2 to 4

The following comparative compositions containing the Agent were prepared as a mixture using the same method described above for Examples 3 to 13

### Comparative Example 2

Agent 250mg
Cellulose acetate phthalate 8.16mg

### Comparative Example 3

Agent 250mg
Ethylcellulose 8.16mg

### Comparative Example 4

Agent 250mg
Cellulose Acetate 8.16mg

### Control Sample

A control sample consisting of 250mg of the Agent alone was prepared for comparison with the compositions of Examples 3 to 13 above.

The excipients used in Examples 3 to 14 and comparative Examples 2 to 4 are all commercially available and had the following characteristics:
Hydroxypropyl methylcellulose was Pharmacoat 606, ex. Shin-Etsu, viscosity 6cP (2w/v% aqueous solution at 20°C as measured by USP method USP 24, NF 19, 2000, p843-844 and USP 24, NF 19, 2000 p2002-2003).
   Benzalkonium chloride was ex Fluka.
   Methylcellulose was Methocel MC, viscosity 10-25cP (2%w/v solution in water at 20°C), ex Fluka.
   Hydroxypropyl cellulose was ex Aldrich, average MW 370 000. Viscosity 150-400cP (Brookfield spindle #2, 60rpm, 2% w/v in water, 25°C).
   Sodium carboxymethylcellulose was Luxara 1316PA (ex. Arthur Branwell & Co Ltd). Degree of substitution 0.8 - 0.95. Viscosity 900-1500cP (1%w/v solution).
   Cellulose acetate phthalate was ex. Aldrich, viscosity specification 45-90cP at 25°C, actual sample viscosity 66.20cP at 25°C.
   Ethylcellulose was ex Fisher.
   Cellulose Acetate was ex Fisher, viscosity 75-100cP (6%w/v solution in 95% acetone/water at 25°C).
   HPMC acetate succinate was Aqoat AS-LG ex. Shin-Etsu. Kinematic viscosity 2.4 - 3.6mm²/s (measured using the method described in Japanese Pharmaceutical Excipients 1993).
   HPMC phthalate was HP55 ex. Shin-Etsu. Viscosity 4x10⁻⁵ m²s⁻¹ (10wt% in a mixture of equal weights of methanol and methylene chloride, measured according to the USP/NF method described in USP 24, NF 19, 2000 p2002-2003).
   Hydroxyethylcellulose was ex. Aldrich. Viscosity spec. 80-125cP (2%w/v solution in water at 25°C. Actual viscosity 107cP. Mw approximately 250 000.

The compositions of Examples 3 to 14 and Comparative Examples 2 to 4 were tested using an analogous pH shift dissolution test to that described in Examples 1 and 2 except that:
(i) Each composition was weighed into a vial and added to 450ml of the acidic dissolution medium. The vial was then rinsed with a further 50ml of fresh acidic medium and the washings added to the main dissolution medium to ensure complete transfer of the composition (total volume of acid medium = 500ml).
(ii) In Examples 7 and 13, the Example composition was dissolved in 50ml of acidic medium in a volumetric flask and the resulting solution was transferred to the dissolution vessel containing 400ml of the acidic dissolution medium. The flask was rinsed with a further 50ml of acidic medium to ensure complete transfer of the composition to the dissolution vessel. This method was also used to measure the pH shift dissolution profile for the Control Sample containing 250mg of the Agent alone.
(iii) In Example 14, for convenience, the benzalkonium chloride was added to the dissolution vessel containing the Agent and HPMC in the acidic dissolution medium (see description of Example 14).
(iv) For Examples 3, 5, 9 and 14 and the control sample (Agent alone) samples of the dissolution medium were removed at 60 minutes (acidic media of approximate pH 1.5 to determine the amount of Agent in solution prior to pH shift). Further samples were then taken up to 60 minutes post pH adjustment as described in Examples 1 and 2. For the remaining Examples and Comparative Examples, sampling was stopped 5 minutes after adjusting the pH from 1.5 to 6.5.

### Results

### Effect of Water-Soluble Cellulose Ethers

Table 1 shows the results from the pH shift dissolution test for the Examples described above which contained the Agent (250mg) and 8.16mg of a water-soluble cellulose ether or an ester of a water-soluble cellulose ether. Also in Table 1 are the results from a control sample of the Agent alone and data from Comparative Examples 2 to 4. In Table 1 the third column indicates the % of the Agent that has dissolved in the acidic dissolution medium after 60 minutes (those cases that indicate >100% result from the experimental error associated with the HPLC analysis). Columns 4 and 5 show the % Agent in solution at 2 and 5 minutes following shift of pH to 6.5.

**Table 1**

| **Example** | **Composition** | **% Agent in solution after 60 mins in acidic media (∼pH 1.5)** | **% Agent in solution at 2 mins after pH adjustment to pH 6.5** | **% Agent in solution at 5 mins after pH adjustment to pH 6.5** |
|---|---|---|---|---|
| 3 | Agent, Methylcellulose | 102.7 | 82.6 | 71.3 |
| 4 | Agent, Sodium Carboxymethylcellulose | 101.0 | 42.9 | 48.3 |
| 5 | Agent, Hydroxypropylcellulose | 103.7 | 78.5 | 30.7 |
| 6 | Agent, Hydroxyethylcellulose | 102.2 | 41.1 | 19.0 |
| 7 | Agent, Hydroxypropyl methylcellulose | 99.5 | 24.6 | 18.3 |
| Comparative 2 | Agent, Cellulose acetate phthalate | 104.4 | 16.8 | 8.0 |
| Comparative 3 | Agent, Ethylcellulose | 101.4 | 15.3 | 7.0 |
| Comparative 4 | Agent, Cellulose Acetate | 103.4 | 11.1 | 6.5 |
| Control | Agent alone | 102.6 | 6.5 | 6.9 |

Table 1 clearly shows that Examples 3 to 7 according to the present invention inhibit precipitation of the Agent from solution upon shift to pH 6.5 compared to the control sample containing the Agent alone. In Example 3 (methylcellulose + Agent) 71.3% of the Agent was still in solution 5 minutes after shift to pH 6.5 compared to just 6.9% of the control sample. Table 1 also shows that the compositions of Comparative Examples 2 to 4 which contain water-insoluble cellulose derivatives, have no significant effect upon inhibiting precipitation of the Agent compared to the control sample of the Agent alone.

Figure 2 illustrates the marked effect upon reduction of precipitation of the Agent of Examples 3 and 5 compared to the control sample of the Agent alone. Figure 2 shows that precipitation of the Agent is significantly reduced even at 60 minutes following pH shift to 6.5 using a relatively small amount of water-soluble cellulose ether compared to the Agent alone. 60 minutes after shifting the pH to 6.5, approximately 15% of the Agent remained in solution for Examples 3 and 5 (compositions containing the Agent and methyl cellulose or hydroxypropylcellulose), whereas less than 5% of the Agent was in solution for the control sample of the Agent alone.

Table 2 shows the effect of increasing the amount of the water-soluble cellulose ether, HPMC, in compositions containing the Agent, on the rate of precipitation of the Agent from solution following pH shift to pH 6.5.

**Table 2**

| **Example** | **Composition** | **% Agent in solution after 60 mins in acidic media ∼pH 1.5** | **% Agent in solution at 2 mins after pH adjustment to pH 6.5** | **% in solution at 5 mins after pH adjustment to pH 6.5** |
|---|---|---|---|---|
| 8 | Agent (250mg), Hydroxypropyl methylcellulose (100mg) | 96.9 | 69.7 | 56.3 |
| 9 | Agent (250mg), Hydroxypropyl methylcellulose (75mg) | 90.0 | 85.6 | 79.5 |
| 10 | Agent (250mg), Hydroxypropyl methylcellulose (25mg) | 98.8 | 67.5 | 55.0 |
| 7 | Agent (250mg), Hydroxypropyl methylcellulose (8.16mg) | 99.5 | 24.6 | 18.3 |
| Control | Agent alone (250mg) | 102.6 | 6.5 | 6.9 |

Table 2 shows that a marked reduction in the rate of precipitation of the Agent is observed with increasing levels of HPMC in the composition. A particularly significant reduction in precipitation of the Agent was obtained with the composition of Example 9 (weight ratio of Agent to HPMC of approximately 3.3:1).

Figure 3 compares the pH shift dissolution profiles for Examples 7 and 9 with the control sample containing the Agent alone. Figure 3 clearly shows that increasing the HPMC level significantly inhibits precipitation of the Agent compared to the Control sample containing the Agent alone.

### Effect of Esters of Water-Soluble Cellulose Ethers

Table 3 compares the pH shift dissolution data of the Agent alone (control) with that of the compositions containing the Agent and an ester of water-soluble cellulose ethers of Examples 11 and 12 enteric polymer. Table 3 also shows the data obtained from Comparative Examples 2 and 4 which contain water-insoluble cellulose esters. In Table 3 for each composition the Agent content is 250mg and the excipient content is 8.16mg.

**Table 3**

| **Example** | **Composition** | **% Agent in solution after 60 mins in acidic media** ∼**pH 1.5** | **% Agent in solution at 2 mins after pH adjustment to pH 6.5** | **% Agent in solution at 5 mins after pH adjustment to pH 6.5** |
|---|---|---|---|---|
| 11 | Agent, Hydroxypropyl methylcellulose acetate succinate | 103.9 | 39.2 | 22.8 |
| 12 | Agent, Hydroxypropyl methylcellulose phthalate | 104.1 | 26.4 | 11.9 |
| Comparative 2 | Agent, Cellulose acetate phthalate | 104.4 | 16.8 | 8.0 |
| Comparative 3 | Agent, Cellulose Acetate | 103.4 | 11.1 | 6.5 |
| Control | Agent | 102.6 | 6.5 | 6.9 |

Table 3 clearly shows that the compositions according to the invention containing an ester of a water-soluble cellulose ether significantly reduce the rate of precipitation of the Agent compared to the Agent alone or the compositions of the Comparative Examples.

### Effect of Wetting Agents

Table 4 compares the pH shift dissolution data for the compositions of Examples 13 and 14 which contain a wetting agent and a water-soluble cellulose ether, with the same composition without a wetting agent (Example 7). Table 4 also contains the data for the control sample of the Agent alone. In Table 3 for each composition shown the Agent content is 250mg and the hydroxypropyl methylcellulose content is 8.16mg and in Examples 13 and 14 the wetting agent content is 1.5mg.

**Table 4**

| **Example** | **Composition** | **% Agent in solution after 60 mins in acidic media ∼pH 1.5** | **% Agent in solution at 2 mins after pH adjustment to pH 6.5** | **% Agent in solution at 5 mins after pH adjustment to pH 6.5** |
|---|---|---|---|---|
| 14 | Agent, Hydroxypropyl methylcellulose, Benzalkonium Chloride | 102.9 | 68.2 | 44.2 |
| 13 | Agent, Hydroxypropyl methylcellulose, Sodium Lauryl Sulphate | 101.4 | 41.6 | 21.9 |
| 7 | Agent, Hydroxypropyl methylcellulose | 99.5 | 24.6 | 18.3 |
| Control | Agent | 102.6 | 6.5 | 6.9 |

Table 4 shows that inclusion of a wetting agent in the composition together with a water-soluble cellulose ether has a marked effect upon reducing the rate of precipitation of the Agent following pH shift to 6.5. In particular the inclusion of a cationic surfactant such as benzalkonium chloride in the composition results in a surprising reduction in the rate of precipitation of the Agent._

Figure 4 further illustrates the effect of including a wetting agent in the compositions according to the present invention. Figure 4 shows the marked reduction on precipitation of the Agent when the composition of Example 14 was used which contained HPMC and the cationic surfactant benzalkonium chloride compared to the use of HPMC alone or to the Control sample containing the Agent alone.

## Claims

1. A pharmaceutical composition comprising 4-(3'-chloro-4'-fluoroanilino)-7-methoxy-6-(3-morpholinopropoxy)quinazoline or a pharmaceutically-acceptable salt thereof (the "Agent") and a water-soluble cellulose ether or an ester of a water-soluble cellulose ether.

2. A pharmaceutical composition according to claim 1 comprising the Agent and a water-soluble cellulose ether wherein the water-soluble cellulose ether is selected from hydroxyethylcellulose, hydroxypropylcellulose, hydroxypropyl methylcellulose and a water-soluble salt of carboxymethylcellulose.

3. A pharmaceutical composition according to claim 1 comprising the Agent and an ester of a water-soluble cellulose ether wherein the ester of a water-soluble cellulose ether is an ester of hydroxypropyl methylcellulose or hydroxypropyl cellulose which carries one or more ester groups selected from acetate, succinate, phthalate, isophthalate, terephthalate and trimellitate.

4. A pharmaceutical composition according to claim 1 wherein the water-soluble cellulose ether or ester of a water-soluble cellulose ether is selected from hydroxypropyl cellulose, hydroxyethylcellulose, methylcellulose, sodium carboxymethylcellulose and hydroxypropyl methylcellulose acetate succinate.

5. A pharmaceutical composition according to claim 1 comprising the Agent and hydroxypropyl methylcellulose.

6. A pharmaceutical composition according to either claim 1 or claim 2 wherein the water-soluble cellulose ether is not hydroxypropyl methylcellulose.

7. A pharmaceutical composition according to any one of the preceding claims wherein the weight ratio of the Agent to water-soluble cellulose ether and ester of a water-soluble cellulose ether is from 40:1 to 2.5:1.

8. A pharmaceutical composition according to any one of the preceding claims further comprising a wetting agent.

9. A pharmaceutical composition according to claim 8 wherein the wetting agent is selected from a pharmaceutically acceptable cationic or anionic surfactant.

10. A pharmaceutical composition according to claim 8 wherein the wetting agent is an alkali metal (8-20C)alkyl sulphate.

11. A pharmaceutical composition according to any one of the preceding claims comprising the Agent, a water-soluble cellulose ether and/or ester of a water-soluble cellulose ether, a wetting agent and one or more fillers, binders, disintegrants or lubricants.

12. A pharmaceutical composition comprising:
(a) from 10 to 80 parts of 4-(3'-chloro-4'-fluoroanilino)-7-methoxy-6-(3-morpholinopropoxy)quinazoline or a pharmaceutically-acceptable salt thereof (the Agent);
(b) from 0.05 to 5 parts wetting agent selected from an anionic surfactant;
(c) from 10 to 60 parts of one or more fillers selected from lactose, mannitol and microcrystalline cellulose;
(d) from 1 to 10 parts of one or more disintegrants selected from carboxymethylcellulose sodium, carboxymethylcellulose calcium, croscarmellose sodium, crospovidone and sodium starch glycolate ;
(e) from 1 to 20 parts of a binder selected from a polyvinylpyrrolidone and hydroxypropyl methylcellulose; and
(f) 0 to 3 parts of a lubricant;
wherein all parts are by weight and the sum of the parts (a)+(b)+(c)+(d)+(e)+(f)=100, and wherein at least one of the components selected from (d) or (e) contains a water-soluble cellulose ether selected from hydroxypropyl methylcellulose and carboxymethylcellulose sodium.

13. A pharmaceutical composition according to any one of the preceding claims which is a solid pharmaceutical composition adapted for oral administration.

14. A solid pharmaceutical composition comprising:
(i) a core comprising 4-(3'-chloro-4'-fluoroanilino)-7-methoxy-6-(3-morpholinopropoxy)quinazoline or a pharmaceutically-acceptable salt thereof (the Agent); and
(ii) a coating comprising an ester of a water-soluble cellulose ether or a water-soluble cellulose ether.

15. A solid pharmaceutical composition according to claim 14 which is a tablet, pellet or granule adapted for oral administration, comprising a core coated with a film coating wherein:
the core comprises:
from 45 to 55% of the Agent;
from 25 to 40% lactose;
from 5 to 15% microcrystal line cellulose;
from 2 to 6% disintegrant;
from 1 to 5% povidone;
from 0.05 tol% sodium dodecyl sulphate; and
from 0.1 to 4% lubricant;
and wherein the film coating comprises:
from 0.5 to 3% water-soluble cellulose ether;
from 0 to 0.5% plasticiser;
from 0 to 0.5% dispersion aid;
from 0 to 0.5% opacifier; and
from 0 to 0.5% colorant;
wherein all % are by weight based upon the total weight of the composition.

16. A pharmaceutical composition according to any one of the preceding claims wherein the Agent is 4-(3'-chloro-4'-fluoroanilino)-7-methoxy-6-(3-morpholinopropoxy)quinazoline.

17. A method of preparing a pharmaceutical composition which comprises admixing 4-(3'-chloro-4'-fluoroanilino)-7-methoxy-6-(3-morpholinopropoxy)quinazoline or a pharmaceutically-acceptable salt thereof with a water-soluble cellulose ether and/or or ester of a water-soluble cellulose ether.

18. The use of a composition according to any one of claims 1 to 16 in the manufacture of a medicament for inhibiting the rate of precipitation of 4-(3'-chloro-4'-fluoroanilino)-7-methoxy-6-(3-morpholinopropoxy)quinazoline or a pharmaceutically-acceptable salt thereof from solution in the GI tract of a warm-blooded mammal.

19. The use of a pharmaceutical composition according to any one of claims 1 to 16 in the manufacture of a medicament for reducing inter-patient variability in bioavailability and/or plasma concentrations of 4-(3'-chloro-4'-fluoroanilino)-7-methoxy-6-(3-morpholinopropoxy)quinazoline or a pharmaceutically-acceptable salt thereof.

20. The use of a water-soluble cellulose ether or an ester of a water-soluble cellulose ether to inhibit the precipitation of the Agent from an aqueous solution, wherein the Agent is as defined in claim 1.

21. A pharmaceutical composition according to any one of claims 1 to 16 for use in the treatment of a cancer.

## Patentansprüche

1. Pharmazeutische Zusammensetzung, enthaltend 4-(3'-Chlor-4'-fluoranilino)-7-methoxy-6-(3-morpholinopropoxy)chinazolin oder ein pharmazeutisch annehmbares Salz davon (das "Mittel") und einen wasserlöslichen Celluloseether oder einen Ester eines wasserlöslichen Celluloseethers.

2. Pharmazeutische Zusammensetzung nach Anspruch 1, enthaltend das Mittel und einen wasserlöslichen Celluloseether, wobei der wasserlösliche Celluloseether ausgewählt ist aus Hydroxyethylcellulose, Hydroxypropylcellulose, Hydroxypropylmethylcellulose und einem wasserlöslichen Salz von Carboxymethylcellulose.

3. Pharmazeutische Zusammensetzung nach Anspruch 1, enthaltend das Mittel und einen Ester eines wasserlöslichen Celluloseethers, wobei es sich bei dem Ester eines wasserlöslichen Celluloseethers um einen Ester von Hydroxypropylmethylcellulose oder Hydroxypropylcellulose handelt, der eine oder mehrere Estergruppen ausgewählt aus Acetat, Succinat, Phthalat, Isophthalat, Terephthalat und Trimellitat trägt.

4. Pharmazeutische Zusammensetzung nach Anspruch 1, wobei der wasserlösliche Celluloseether oder der Ester eines wasserlöslichen Celluloseethers ausgewählt ist aus Hydroxypropylcellulose, Hydroxyethylcellulose, Methylcellulose, Natriumcarboxymethylcellulose und Hydroxypropylcelluloseacetatsuccinat.

5. Pharmazeutische Zusammensetzung nach Anspruch 1, enthaltend das Mittel und Hydroxypropylmethylcellulose.

6. Pharmazeutische Zusammensetzung nach Anspruch 1 oder 2, wobei es sich bei dem wasserlöslichen Celluloseether nicht um Hydroxypropylmethylcellulose handelt.

7. Pharmazeutische Zusammensetzung nach einem der vorhergehenden Ansprüche, wobei das Gewichtsverhältnis des Mittels zum wasserlöslichen Celluloseether und Ester eines wasserlöslichen Celluloseethers 40:1 bis 2,5:1 beträgt.

8. Pharmazeutische Zusammensetzung nach einem der vorhergehenden Ansprüche, weiterhin enthaltend ein Netzmittel.

9. Pharmazeutische Zusammensetzung nach Anspruch 8, wobei das Netzmittel ausgewählt ist aus einem pharmazeutisch annehmbaren kationischen oder anionischen Tensid.

10. Pharmazeutische Zusammensetzung nach Anspruch 8, wobei es sich bei dem Netzmittel um ein Alkali-(8-20C)-Alkylsulfat handelt.

11. Pharmazeutische Zusammensetzung nach einem der vorhergehenden Ansprüche, enthaltend das Mittel, einen wasserlöslichen Celluloseether und/oder Ester eines wasserlöslichen Celluloseethers, ein Näßmittel und ein oder mehrere Füllstoffe, Bindemittel, Sprengmittel oder Schmiermittel.

12. Pharmazeutische Zusammensetzung, enthaltend:
(a) 10 bis 80 Teile an 4-(3'-Chlor-4'-fluoranilino)-7-methoxy-6-(3-morpholinopropoxy)chinazolin oder einem pharmazeutisch annehmbaren Salz davon (das Mittel);
(b) 0,05 bis 5 Teile eines Netzmittels ausgewählt aus einem anionischen Tensid;
(c) 10 bis 60 Teile eines oder mehrerer Füllstoffe ausgewählt aus Lactose, Manit und mikrokristalliner Cellulose;
(d) 1 bis 10 Teile eines oder mehrerer Sprengmittel ausgewählt aus Carboxymethylcellulose-Natrium, Carboxymethylcellulose-Kalzium, Croscarmellose-Natrium, Crospovidon und Natrium-Stärkeglykolat;
(e) 1 bis 20 Teile eines Bindemittels ausgewählt aus einem Polyvinylpyrrolidon und Hydroxypropylmethylcellulose; und
(f) 0 bis 3 Teile eines Schmiermittels;
wobei alle Teile jeweils Gewichtsteile sind und die Summe der Teile (a)+(b)+(c)+(d)+(e)+(f)=100, und wobei wenigstens eine der Komponenten ausgewählt aus (d) oder (e) einen wasserlöslichen Celluloseether ausgewählt aus Hydroxypropylmethylcellulose und Carboxymethylcellulose-Natrium enthält.

13. Pharmazeutische Zusammensetzung nach einem der vorhergehenden Ansprüche, bei der es sich um eine für die orale Verabreichung ausgelegte feste pharmazeutische Zusammensetzung handelt.

14. Feste pharmazeutische Zusammensetzung, enthaltend:
(i) einen Kern enthaltend 4-(3'-Chlor-4'-fluoranilino)-7-methoxy-6-(3-morpholinopropoxy)chinazolin oder ein pharmazeutisch annehmbares Salz davon (das Mittel);
und
(ii) einen Überzug enthaltend einen Ester eines wasserlöslichen Celluloseethers oder einen wasserlöslichen Celluloseether.

15. Feste pharmazeutische Zusammensetzung nach Anspruch 14, bei der es sich um eine für die orale Verabreichung ausgelegte Tablette, ein für die orale Verabreichung ausgelegtes Pellet oder ein für die orale Verabreichung ausgelegtes Granulat handelt, enthaltend einen mit einem Filmüberzug beschichteten Kern, wobei:
der Kern:
45 bis 55% an Mittel;
25 bis 40% Lactose;
5 bis 15% mikrokristalline Cellulose;
2 bis 6% Sprengmittel;
1 bis 5% Povidon;
0,05 bis 1% Natriumdodecylsulfat; und
0,1 bis 4% Schmiermittel
enthält;
und wobei der Filmüberzug:
0,5 bis 3% an wasserlöslichem Celluloseether;
0 bis 0,5% Weichmacher;
0 bis 0,5% Dispergiermittel;
0 bis 0,5% Trübungsmittel; und
0 bis 0,5% Farbstoff
enthält;
wobei alle Gew.-% auf der Basis des Gesamtgewichts der Zusammensetzung sind.

16. Pharmazeutische Zusammensetzung nach einem der vorhergehenden Ansprüche, wobei es sich bei dem Mittel um 4-(3'-Chlor-4'-fluoranilino)-7-methoxy-6-(3-morpholinopropoxy)chinazolin handelt.

17. Verfahren zur Herstellung einer pharmazeutischen Zusammensetzung, bei dem man 4-(3'-Chlor-4'-fluoranilino)-7-methoxy-6-(3-morpholinopropoxy)chinazolin oder ein pharmazeutisch annehmbares Salz davon mit einem wasserlöslichen Celluloseether und/oder einem Ester eines wasserlöslichen Celluloseethers mischt.

18. Verwendung einer Zusammensetzung nach einem der Ansprüche 1 bis 16 bei der Herstellung eines Medikaments zur Inhibierung der Ausfällungsgeschwindigkeit von 4-(3'-Chlor-4'-fluoranilino)-7-methoxy-6-(3-morpholinopropoxy)chinazolin oder eines pharmazeutisch annehmbaren Salzes davon aus einer Lösung im Magen-Darm-Trakt eines Warmblüters.

19. Verwendung einer pharmazeutischen Zusammensetzung nach einem der Ansprüche 1 bis 16 bei der Herstellung eines Medikaments zur Verminderung der Variabilität der biologischen Verfügbarkeit und/oder der Plasmakonzentrationen von 4-(3'-Chlor-4'-fluoranilino)-7-methoxy-6-(3-morpholinopropoxy)chinazolin oder einem pharmazeutisch annehmbaren Salz davon zwischen Patienten.

20. Verwendung eines wasserlöslichen Celluloseethers oder eines Esters eines wasserlöslichen Celluloseethers zur Inhibierung der Ausfällung des Mittels aus einer wäßrigen Lösung, wobei das Mittel wie in Anspruch 1 definiert ist.

21. Pharmazeutische Zusammensetzung nach einem der Ansprüche 1 bis 16 zur Verwendung bei der Behandlung von Krebs.

## Revendications

1. Composition pharmaceutique comprenant de la 4-(3'-chloro-4'-fluoroanilino)-7-méthoxy-6-(3-morpholinopropoxy)quinazoline ou un sel pharmaceutiquement acceptable de celle-ci (l'Agent) ainsi qu'un éther de cellulose soluble dans l'eau ou un ester d'un éther de cellulose soluble dans l'eau.

2. Composition pharmaceutique selon la revendication 1 comprenant l'Agent et un éther de cellulose soluble dans l'eau, dans laquelle l'éther de cellulose soluble dans l'eau est choisi parmi l'hydroxyéthylcellulose, l'hydroxypropylcellulose, l'hydroxypropylméthylcellulose et un sel soluble dans l'eau de carboxyméthylcellulose.

3. Composition pharmaceutique selon la revendication 1 comprenant l'Agent et un ester d'un éther de cellulose soluble dans l'eau, dans laquelle l'ester d'un éther de cellulose soluble dans l'eau est un ester d'hydroxypropylméthylcellulose ou d'hydroxypropylcellulose qui porte un groupe ester ou plus choisi parmi l'acétate, le succinate, le phtalate, l'isophtalate, le téréphtalate et le trimellitate.

4. Composition pharmaceutique selon la revendication 1, dans laquelle l'éther de cellulose soluble dans l'eau ou l'ester d'un éther de cellulose soluble dans l'eau est choisi parmi l'hydroxypropylcellulose, l'hydroxyéthylcellulose, la méthylcellulose, la carboxyméthylcellulose sodique et l'acétate succinate d'hydroxypropylméthylcellulose.

5. Composition pharmaceutique selon la revendication 1 comprenant l'Agent et de l'hydroxypropylméthylcellulose.

6. Composition pharmaceutique selon l'une des revendications 1 et 2, dans laquelle l'éther de cellulose soluble dans l'eau n'est pas l'hydroxypropylméthylcellulose.

7. Composition pharmaceutique selon l'une quelconque des revendications précédentes, dans laquelle le rapport de poids de l'Agent à l'éther de cellulose soluble dans l'eau et l'ester d'un éther de cellulose soluble dans l'eau varie de 40:1 à 2,5:1.

8. Composition pharmaceutique selon l'une quelconque des revendications précédentes comprenant en outre un agent mouillant.

9. Composition pharmaceutique selon la revendication 8, dans laquelle l'agent mouillant est choisi parmi un agent tensioactif cationique ou anionique pharmaceutiquement acceptable.

10. Composition pharmaceutique selon la revendication 8, dans laquelle l'agent mouillant est un sulfate d'alkyl (8 à 20 atomes de carbone) de métal alcalin.

11. Composition pharmaceutique selon l'une quelconque des revendications précédentes comprenant l'Agent, un éther de cellulose soluble dans l'eau et/ou un ester d'un éther de cellulose soluble dans l'eau, un agent mouillant et un ou plusieurs éléments parmi des matières de charge, des liants, des délitants ou des lubrifiants.

12. Composition pharmaceutique comprenant :
(a) de 10 à 80 parties de 4-(3'-chloro-4'-fluoroanilino)-7-méthoxy-6-(3-morpholinopropoxy)quinazoline ou d'un sel pharmaceutiquement acceptable de celle-ci (l'Agent) ;
(b) de 0,05 à 5 parties d'agent mouillant choisi à partir d'un agent tensioactif anionique ;
(c) de 10 à 60 parties d'une matière de charge ou plus choisie parmi le lactose, le mannitol et la cellulose microcristalline ;
(d) de 1 à 10 parties d'un délitant ou plus choisi parmi la carboxyméthylcellulose sodique, la carboxyméthylcellulose calcique, la croscarmellose sodique, la crospovidone et le glycolate sodique d'amidon ;
(e) de 1 à 20 parties d'un liant choisi parmi la polyvinylpyrrolidone et l'hydroxypropylméthylcellulose ; et
(f) de 0 à 3 parties d'un lubrifiant ;
dans laquelle toutes les parties sont en poids et la somme des parties (a) + (b) + (c) + (d) + (e) + (f) = 100, et dans laquelle au moins un des composants choisi parmi (d) ou (e) comprend un éther de cellulose soluble dans l'eau choisi parmi l'hydroxypropylméthylcellulose et la carboxyméthylcellulose sodique.

13. Composition pharmaceutique selon l'une quelconque des revendications précédentes, laquelle est une composition pharmaceutique solide adaptée pour une administration par voie orale.

14. Composition pharmaceutique solide comprenant :
(i) un noyau comprenant de la 4-(3'-chloro-4'-fluoroanilino)-7-méthoxy-6-(3-morpholinopropoxy)quinazoline ou un sel pharmaceutiquement acceptable de celle-ci (l'Agent);
et
(ii) un revêtement comprenant un ester d'un éther de cellulose soluble dans l'eau ou un éther de cellulose soluble dans l'eau.

15. Composition pharmaceutique solide selon la revendication 14, laquelle est un comprimé, une pastille ou un granule adapté pour une administration par voie orale, comprenant un noyau recouvert d'un enrobage en film, dans laquelle le noyau comprend :
de 45 à 55 % de l'Agent;
de 25 à 40 % de lactose;
de 5 à 15 % de cellulose microcristalline ;
de 2 à 6 % de délitant;
de 1 à 5 % de povidone ;
de 0,05 à 1 % de dodécylsulfate de sodium; et
de 0,1 à 4 % de lubrifiant;
et dans laquelle l'enrobage en film comprend :
de 0,5 à 3 % d'éther de cellulose soluble dans l'eau ;
de 0 à 0,5 % de plastifiant;
de 0 à 0,5 % d'un auxiliaire de dispersion ;
de 0 à 0,5 % d'un opacifiant ; et
de 0 à 0,5 % d'un colorant ;
dans laquelle tous les pourcentages sont en poids basé sur le poids total de la composition.

16. Composition pharmaceutique selon l'une quelconque des revendications précédentes, dans laquelle l'Agent est la 4-(3'-chloro-4'-fluoroanilino)-7-méthoxy-6-(3-morpholinopropoxy)quinazoline.

17. Procédé de préparation d'une composition pharmaceutique qui comprend le mélange de 4-(3'-chloro-4'-fluoroanilino)-7-méthoxy-6-(3-morpholinopropoxy)quinazoline ou d'un sel pharmaceutiquement acceptable de celle-ci avec un éther de cellulose soluble dans l'eau et/ou un ester d'un éther de cellulose soluble dans l'eau.

18. Utilisation d'une composition selon l'une quelconque des revendications 1 à 16 dans la fabrication d'un médicament pour inhiber le taux de précipitation d'une solution de 4-(3'-chloro-4'-fluoroanilino)-7-méthoxy-6-(3-morpholinopropoxy)quinazoline ou d'un sel pharmaceutiquement acceptable de celle-ci dans le tractus gastro-intestinal d'un mammifère à sang chaud.

19. Utilisation d'une composition pharmaceutique selon l'une quelconque des revendications 1 à 16 dans la fabrication d'un médicament qui vise à réduire la variabilité interpatient de la biodisponibilité et/ou des concentrations plasmiques de 4-(3'-chloro-4'-fluoroanilino)-7-méthoxy-6-(3-morpholinopropoxy)quinazoline ou d'un sel pharmaceutiquement acceptable de celle-ci.

20. Utilisation d'un éther de cellulose soluble dans l'eau ou d'un ester d'un éther de cellulose soluble dans l'eau pour inhiber la précipitation de l'Agent à partir d'une solution aqueuse, dans laquelle l'Agent est tel que défini dans la revendication 1.

21. Composition pharmaceutique selon l'une quelconque des revendications 1 à 16 pour une utilisation dans le traitement d'un cancer.
